# EUROPEAN PATENT SPECIFICATION

(11) **EP 2 457 089 B1**
(45) Date of publication and mention of the grant of the patent: **07.08.2019**
(21) Application number: 10747679.8
(22) Date of filing: 21.07.2010
(51) Int. Cl.: G01N 33/50

(54) **A METHOD OF DIAGNOSING CANCER**
VERFAHREN ZUR DIAGNOSE VON KREBS
MÉTHODE DE DIAGNOSTIC DU CANCER

(30) Priority: 21.07.2009 US 227130 P
(43) Date of publication of application: 30.05.2012
(73) Proprietor: Tel HaShomer Medical Research Infrastructure and Services Ltd., 52 621 Ramat Gan (IL)
(72) Inventor: MARKEL, Gal, 62264 Tel-Aviv (IL)
(74) Representative: Becker Kurig Straus
(86) International application number: PCT/IL2010/000581
(87) International publication number: WO 2011/010309

(56) References cited:
- US-A1- 2007 110 668
- GRAY-OWEN SCOTT D ET AL: "CEACAM1: contact-dependent control of immunity" June 2006 (2006-06), NATURE REVIEWS IMMUNOLOGY, VOL. 6, NR. 6, PAGE(S) 433-446 , XP002605706 ISSN: 1474-1733 page 439, column 1, paragraph 2 - column 2, paragraph 4 * abstract
- KAMMERER R ET AL: "The tumour suppressor gene CEACAMI is completely but reversibly downregulated in renal cell carcinoma" JOURNAL OF PATHOLOGY 200411 GB LNKD- DOI:10.1002/PATH.1657, vol. 204, no. 3, November 2004 (2004-11), pages 258-267, XP002605707 ISSN: 0022-3417
- MARKEL GAL ET AL: "CD66a interactions between human melanoma and NK cells: A novel class I MHC-independent inhibitory mechanism of cytotoxicity" 15 March 2002 (2002-03-15), JOURNAL OF IMMUNOLOGY, AMERICAN ASSOCIATION OF IMMUNOLOGISTS, US, PAGE(S) 2803 - 2810 , XP002560494 ISSN: 0022-1767 * abstract
- STERN-GINOSSAR NOAM ET AL: "Intercellular transfer of carcinoembryonic antigen from tumor cells to NK cells" JOURNAL OF IMMUNOLOGY, vol. 179, no. 7, October 2007 (2007-10), pages 4424-4434, XP002605708 ISSN: 0022-1767
- OBRINK ET AL: "On the role of CEACAM1 in cancer" 1 June 2008 (2008-06-01), LUNG CANCER, ELSEVIER, AMSTERDAM, NL LNKD- DOI:10.1016/J.LUNGCAN.2008.03.020, PAGE(S) 309 - 312 , XP022690998 ISSN: 0169-5002 [retrieved on 2008-05-07] page 310, column 2, paragraph 3 - page 311
- MOELLER M J ET AL: "BILIARY GLYCOPROTEIN (BGP) EXPRESSION ON T CELLS AND ON A NATURAL-KILLER-CELL SUB-POPULATION" 1 January 1996 (1996-01-01), INTERNATIONAL JOURNAL OF CANCER, JOHN WILEY & SONS, INC, UNITED STATES, SWITZERLAND, GERMANY LNKD- DOI:10.1002/(SICI)1097-0215(19960315)65:6< 740::AID-IJC5>3.0.CO;2-Z, PAGE(S) 740 - 745 , XP009004617 ISSN: 0020-7136 * abstract

### SEQUENCE LISTING

<110> Tel HaShomer Medical Research Infrastructure and Services Ltd. Markel, Gal
<120> A METHOD OF DIAGNOSING CANCER
<130> 49350
<150> US 61/227,130
   <151> 2009-07-21
<160> 14
<170> PatentIn version 3.5
<210> 1
   <211> 21
   <212> DNA
   <213> Artificial sequence
<220>
   <223> Single strand DNA oligonucleotide
<400> 1
   gagtagtggc cctggttgct c 21
<210> 2
   <211> 17
   <212> DNA
   <213> Artificial sequence
<220>
   <223> Single strand DNA oligonucleotide
<400> 2
   cgctggtcgc ttgccct 17
<210> 3
   <211> 18
   <212> DNA
   <213> Artificial sequence
<220>
   <223> Single strand DNA oligonucleotide
<400> 3
   ggtcctgagc tgccggtc 18
<210> 4
   <211> 3187
   <212> DNA
   <213> Homo sapiens
<400> 4
   aaagctctgg gccccaggga ggaggctcag cacagagagt ggaaaacagc agaggtgaca      60
   gagcagccgt gctcgaagcg ttcctggagc ccaagctctc ctccacaggt gaagacaggg     120
   ccagcaggag acaccatggg gcacctctca gccccacttc acagagtgcg tgtaccctgg     180
   caggggcttc tgctcacagc ctcacttcta accttctgga acccgcccac cactgcccag     240
   ctcactactg aatccatgcc attcaatgtt gcagagggga aggaggttct tctccttgtc     300
   cacaatctgc cccagcaact ttttggctac agctggtaca aaggggaaag agtggatggc     360
   aaccgtcaaa ttgtaggata tgcaatagga actcaacaag ctaccccagg gcccgcaaac     420
   agcggtcgag agacaatata ccccaatgca tccctgctga tccagaacgt cacccagaat     480
   gacacaggat tctacaccct acaagtcata aagtcagatc ttgtgaatga agaagcaact 540
   ggacagttcc atgtataccc ggagctgccc aagccctcca tctccagcaa caactccaac 600
   cctgtggagg acaaggatgc tgtggccttc acctgtgaac ctgagactca ggacacaacc 660
   tacctgtggt ggataaacaa tcagagcctc ccggtcagtc ccaggctgca gctgtccaat 720
   ggcaacagga ccctcactct actcagtgtc acaaggaatg acacaggacc ctatgagtgt 780
   gaaatacaga acccagtgag tgcgaaccgc agtgacccag tcaccttgaa tgtcacctat 840
   ggcccggaca cccccaccat ttccccttca gacacctatt accgtccagg ggcaaacctc 900
   agcctctcct gctatgcagc ctctaaccca cctgcacagt actcctggct tatcaatgga 960
   acattccagc aaagcacaca agagctcttt atccctaaca tcactgtgaa taatagtgga 1020
   tcctatacct gccacgccaa taactcagtc actggctgca acaggaccac agtcaagacg 1080
   atcatagtca ctgataatgc tctaccacaa gaaaatggcc tctcacctgg ggccattgct 1140
   ggcattgtga ttggagtagt ggccctggtt gctctgatag cagtagccct ggcatgtttt 1200
   ctgcatttcg ggaagaccgg cagctcagga ccactccaat gacccaccta acaagatgaa 1260
   tgaagttact tattctaccc tgaactttga agcccagcaa cccacacaac caacttcagc 1320
   ctccccatcc ctaacagcca cagaaataat ttattcagaa gtaaaaaagc agtaatgaaa 1380
   cctgtcctgc tcactgcagt gctgatgtat ttcaagtctc tcaccctcat cactaggaga 1440
   ttcctttccc ctgtaggggt agaggggtgg ggacagaaac aactttctcc tactcttcct 1500
   tcctaatagg catctccagg ctgcctggtc actgcccctc tctcagtgtc aatagatgaa 1560
   agtacattgg gagtctgtag gaaacccaac cttcttgtca ttgaaatttg gcaaagctga 1620
   ctttgggaaa gagggaccag aacttcccct cccttcccct tttcccaacc tggacttgtt 1680
   ttaaacttgc ctgttcagag cactcattcc ttcccacccc cagtcctgtc ctatcactct 1740
   aattcggatt tgccatagcc ttgaggttat gtccttttcc attaagtaca tgtgccagga 1800
   aacaagagag agagaaagta aaggcagtaa tgccttctcc tatttctcca aagccttgtg 1860
   tgaactcacc aaacacaaga aaatcaaata tataaccaat agtgaaatgc cacacctttg 1920
   tccactgtca gggttgtcta cctgtaggat cagggtctaa gcaccttggt gcttagctag 1980
   aataccacct aatccttctg gcaagcctgt cttcagagaa cccactagaa gcaactagga 2040
   aaatcacttg ccaaaatcca aggcaattcc tgatggaaaa tgcaaaagca catatatgtt 2100
   ttaatatctt tatgggctct gttcaaggca gtgctgagag ggaggggtta tagcttcagg 2160
   agggaaccag cttctgataa acacaatctg ctaggaactt gggaaaggaa tcagagagct 2220
   gcccttcagc gattatttaa attattgtta aagaatacac aatttggggt attgggattt 2280
   ttctcctttt ctctgagaca ttccaccatt ttaatttttg taactgctta tttatgtgaa 2340
   aagggttatt tttacttagc ttagctatgt cagccaatcc gattgcctta ggtgaaagaa 2400
   accaccgaaa tccctcaggt cccttggtca ggagcctctc aagatttttt ttgtcagagg 2460
   ctccaaatag aaaataagaa aaggttttct tcattcatgg ctagagctag atttaactca 2520
   gtttctaggc acctcagacc aatcatcaac taccattcta ttccatgttt gcacctgtgc 2580
   attttctgtt tgcccccatt cactttgtca ggaaaccttg gcctctgcta aggtgtattt 2640
   ggtccttgag aagtgggagc accctacagg gacactatca ctcatgctgg tggcattgtt 2700
   tacagctaga aagctgcact ggtgctaatg ccccttgggg aaatggggct gtgaggagga      2760
   ggattataac ttaggcctag cctcttttaa cagcctctga aatttatctt ttcttctatg      2820
   gggtctataa atgtatctta taataaaaag gaaggacagg aggaagacag gcaaatgtac      2880
   ttctcaccca gtcttctaca cagatggaat ctctttgggg ctaagagaaa ggttttattc      2940
   tatattgctt acctgatctc atgttaggcc taagaggctt tctccaggag gattagcttg      3000
   gagttctcta tactcaggta cctctttcag ggttttctaa ccctgacacg gactgtgcat      3060
   actttccctc atccatgctg tgctgtgtta tttaattttt cctggctaag atcatgtctg      3120
   aattatgtat gaaaattatt ctatgttttt ataataaaaa taatatatca gacatcgaaa      3180
   aaaaaaa                                                                3187
<210> 5
   <211> 3240
   <212> DNA
   <213> Homo sapiens
<400> 5
   aaagctctgg gccccaggga ggaggctcag cacagagagt ggaaaacagc agaggtgaca       60
   gagcagccgt gctcgaagcg ttcctggagc ccaagctctc ctccacaggt gaagacaggg      120
   ccagcaggag acaccatggg gcacctctca gccccacttc acagagtgcg tgtaccctgg      180
   caggggcttc tgctcacagc ctcacttcta accttctgga acccgcccac cactgcccag      240
   ctcactactg aatccatgcc attcaatgtt gcagagggga aggaggttct tctccttgtc      300
   cacaatctgc cccagcaact ttttggctac agctggtaca aaggggaaag agtggatggc      360
   aaccgtcaaa ttgtaggata tgcaatagga actcaacaag ctaccccagg gcccgcaaac      420
   agcggtcgag agacaatata ccccaatgca tccctgctga tccagaacgt cacccagaat      480
   gacacaggat tctacaccct acaagtcata aagtcagatc ttgtgaatga agaagcaact      540
   ggacagttcc atgtataccc ggagctgccc aagccctcca tctccagcaa caactccaac      600
   cctgtggagg acaaggatgc tgtggccttc acctgtgaac ctgagactca ggacacaacc      660
   tacctgtggt ggataaacaa tcagagcctc ccggtcagtc ccaggctgca gctgtccaat      720
   ggcaacagga ccctcactct actcagtgtc acaaggaatg acacaggacc ctatgagtgt      780
   gaaatacaga acccagtgag tgcgaaccgc agtgacccag tcaccttgaa tgtcacctat      840
   ggcccggaca cccccaccat ttccccttca gacacctatt accgtccagg ggcaaacctc      900
   agcctctcct gctatgcagc ctctaaccca cctgcacagt actcctggct tatcaatgga      960
   acattccagc aaagcacaca agagctcttt atccctaaca tcactgtgaa taatagtgga     1020
   tcctatacct gccacgccaa taactcagtc actggctgca acaggaccac agtcaagacg     1080
   atcatagtca ctgataatgc tctaccacaa gaaaatggcc tctcacctgg ggccattgct     1140
   ggcattgtga ttggagtagt ggccctggtt gctctgatag cagtagccct ggcatgtttt     1200
   ctgcatttcg ggaagaccgg cagggcaagc gaccagcgtg atctcacaga gcacaaaccc     1260
   tcagtctcca accacactca ggaccactcc aatgacccac ctaacaagat gaatgaagtt     1320
   acttattcta ccctgaactt tgaagcccag caacccacac aaccaacttc agcctcccca     1380
   tccctaacag ccacagaaat aatttattca gaagtaaaaa agcagtaatg aaacctgtcc     1440
   tgctcactgc agtgctgatg tatttcaagt ctctcaccct catcactagg agattccttt 1500
   cccctgtagg ggtagagggg tggggacaga aacaactttc tcctactctt ccttcctaat 1560
   aggcatctcc aggctgcctg gtcactgccc ctctctcagt gtcaatagat gaaagtacat 1620
   tgggagtctg taggaaaccc aaccttcttg tcattgaaat ttggcaaagc tgactttggg 1680
   aaagagggac cagaacttcc cctcccttcc ccttttccca acctggactt gttttaaact 1740
   tgcctgttca gagcactcat tccttcccac ccccagtcct gtcctatcac tctaattcgg 1800
   atttgccata gccttgaggt tatgtccttt tccattaagt acatgtgcca ggaaacaaga 1860
   gagagagaaa gtaaaggcag taatgccttc tcctatttct ccaaagcctt gtgtgaactc 1920
   accaaacaca agaaaatcaa atatataacc aatagtgaaa tgccacacct ttgtccactg 1980
   tcagggttgt ctacctgtag gatcagggtc taagcacctt ggtgcttagc tagaatacca 2040
   cctaatcctt ctggcaagcc tgtcttcaga gaacccacta gaagcaacta ggaaaatcac 2100
   ttgccaaaat ccaaggcaat tcctgatgga aaatgcaaaa gcacatatat gttttaatat 2160
   ctttatgggc tctgttcaag gcagtgctga gagggagggg ttatagcttc aggagggaac 2220
   cagcttctga taaacacaat ctgctaggaa cttgggaaag gaatcagaga gctgcccttc 2280
   agcgattatt taaattattg ttaaagaata cacaatttgg ggtattggga tttttctcct 2340
   tttctctgag acattccacc attttaattt ttgtaactgc ttatttatgt gaaaagggtt 2400
   atttttactt agcttagcta tgtcagccaa tccgattgcc ttaggtgaaa gaaaccaccg 2460
   aaatccctca ggtcccttgg tcaggagcct ctcaagattt tttttgtcag aggctccaaa 2520
   tagaaaataa gaaaaggttt tcttcattca tggctagagc tagatttaac tcagtttcta 2580
   ggcacctcag accaatcatc aactaccatt ctattccatg tttgcacctg tgcattttct 2640
   gtttgccccc attcactttg tcaggaaacc ttggcctctg ctaaggtgta tttggtcctt 2700
   gagaagtggg agcaccctac agggacacta tcactcatgc tggtggcatt gtttacagct 2760
   agaaagctgc actggtgcta atgccccttg gggaaatggg gctgtgagga ggaggattat 2820
   aacttaggcc tagcctcttt taacagcctc tgaaatttat cttttcttct atggggtcta 2880
   taaatgtatc ttataataaa aaggaaggac aggaggaaga caggcaaatg tacttctcac 2940
   ccagtcttct acacagatgg aatctctttg gggctaagag aaaggtttta ttctatattg 3000
   cttacctgat ctcatgttag gcctaagagg ctttctccag gaggattagc ttggagttct 3060
   ctatactcag gtacctcttt cagggttttc taaccctgac acggactgtg catactttcc 3120
   ctcatccatg ctgtgctgtg ttatttaatt tttcctggct aagatcatgt ctgaattatg 3180
   tatgaaaatt attctatgtt tttataataa aaataatata tcagacatcg aaaaaaaaaa 3240
<210> 6
   <211> 3333
   <212> DNA
   <213> Homo sapiens
<400> 6
   aaagctctgg gccccaggga ggaggctcag cacagagagt ggaaaacagc agaggtgaca      60
   gagcagccgt gctcgaagcg ttcctggagc ccaagctctc ctccacaggt gaagacaggg     120
   ccagcaggag acaccatggg gcacctctca gccccacttc acagagtgcg tgtaccctgg     180
   caggggcttc tgctcacagc ctcacttcta accttctgga acccgcccac cactgcccag     240
   ctcactactg aatccatgcc attcaatgtt gcagagggga aggaggttct tctccttgtc 300
   cacaatctgc cccagcaact ttttggctac agctggtaca aaggggaaag agtggatggc 360
   aaccgtcaaa ttgtaggata tgcaatagga actcaacaag ctaccccagg gcccgcaaac 420
   agcggtcgag agacaatata ccccaatgca tccctgctga tccagaacgt cacccagaat 480
   gacacaggat tctacaccct acaagtcata aagtcagatc ttgtgaatga agaagcaact 540
   ggacagttcc atgtataccc ggagctgccc aagccctcca tctccagcaa caactccaac 600
   cctgtggagg acaaggatgc tgtggccttc acctgtgaac ctgagactca ggacacaacc 660
   tacctgtggt ggataaacaa tcagagcctc ccggtcagtc ccaggctgca gctgtccaat 720
   ggcaacagga ccctcactct actcagtgtc acaaggaatg acacaggacc ctatgagtgt 780
   gaaatacaga acccagtgag tgcgaaccgc agtgacccag tcaccttgaa tgtcacctat 840
   ggcccggaca cccccaccat ttccccttca gacacctatt accgtccagg ggcaaacctc 900
   agcctctcct gctatgcagc ctctaaccca cctgcacagt actcctggct tatcaatgga 960
   acattccagc aaagcacaca agagctcttt atccctaaca tcactgtgaa taatagtgga 1020
   tcctatacct gccacgccaa taactcagtc actggctgca acaggaccac agtcaagacg 1080
   atcatagtca ctgagagaca gaatctcacc atgttaccca ggctggactc gaactcctgg 1140
   gctcaagcaa tcctcccatc tgtttcccaa agtgctgaga ttacagataa tgctctacca 1200
   caagaaaatg gcctctcacc tggggccatt gctggcattg tgattggagt agtggccctg 1260
   gttgctctga tagcagtagc cctggcatgt tttctgcatt tcgggaagac cggcagggca 1320
   agcgaccagc gtgatctcac agagcacaaa ccctcagtct ccaaccacac tcaggaccac 1380
   tccaatgacc cacctaacaa gatgaatgaa gttacttatt ctaccctgaa ctttgaagcc 1440
   cagcaaccca cacaaccaac ttcagcctcc ccatccctaa cagccacaga aataatttat 1500
   tcagaagtaa aaaagcagta atgaaacctg tcctgctcac tgcagtgctg atgtatttca 1560
   agtctctcac cctcatcact aggagattcc tttcccctgt aggggtagag gggtggggac 1620
   agaaacaact ttctcctact cttccttcct aataggcatc tccaggctgc ctggtcactg 1680
   cccctctctc agtgtcaata gatgaaagta cattgggagt ctgtaggaaa cccaaccttc 1740
   ttgtcattga aatttggcaa agctgacttt gggaaagagg gaccagaact tcccctccct 1800
   tccccttttc ccaacctgga cttgttttaa acttgcctgt tcagagcact cattccttcc 1860
   cacccccagt cctgtcctat cactctaatt cggatttgcc atagccttga ggttatgtcc 1920
   ttttccatta agtacatgtg ccaggaaaca agagagagag aaagtaaagg cagtaatgcc 1980
   ttctcctatt tctccaaagc cttgtgtgaa ctcaccaaac acaagaaaat caaatatata 2040
   accaatagtg aaatgccaca cctttgtcca ctgtcagggt tgtctacctg taggatcagg 2100
   gtctaagcac cttggtgctt agctagaata ccacctaatc cttctggcaa gcctgtcttc 2160
   agagaaccca ctagaagcaa ctaggaaaat cacttgccaa aatccaaggc aattcctgat 2220
   ggaaaatgca aaagcacata tatgttttaa tatctttatg ggctctgttc aaggcagtgc 2280
   tgagagggag gggttatagc ttcaggaggg aaccagcttc tgataaacac aatctgctag 2340
   gaacttggga aaggaatcag agagctgccc ttcagcgatt atttaaatta ttgttaaaga 2400
   atacacaatt tggggtattg ggatttttct ccttttctct gagacattcc accattttaa 2460
   tttttgtaac tgcttattta tgtgaaaagg gttattttta cttagcttag ctatgtcagc     2520
   caatccgatt gccttaggtg aaagaaacca ccgaaatccc tcaggtccct tggtcaggag     2580
   cctctcaaga ttttttttgt cagaggctcc aaatagaaaa taagaaaagg ttttcttcat     2640
   tcatggctag agctagattt aactcagttt ctaggcacct cagaccaatc atcaactacc     2700
   attctattcc atgtttgcac ctgtgcattt tctgtttgcc cccattcact ttgtcaggaa     2760
   accttggcct ctgctaaggt gtatttggtc cttgagaagt gggagcaccc tacagggaca     2820
   ctatcactca tgctggtggc attgtttaca gctagaaagc tgcactggtg ctaatgcccc     2880
   ttggggaaat ggggctgtga ggaggaggat tataacttag gcctagcctc ttttaacagc     2940
   ctctgaaatt tatcttttct tctatggggt ctataaatgt atcttataat aaaaaggaag     3000
   gacaggagga agacaggcaa atgtacttct cacccagtct tctacacaga tggaatctct     3060
   ttggggctaa gagaaaggtt ttattctata ttgcttacct gatctcatgt taggcctaag     3120
   aggctttctc caggaggatt agcttggagt tctctatact caggtacctc tttcagggtt     3180
   ttctaaccct gacacggact gtgcatactt tccctcatcc atgctgtgct gtgttattta     3240
   atttttcctg gctaagatca tgtctgaatt atgtatgaaa attattctat gtttttataa     3300
   taaaaataat atatcagaca tcgaaaaaaa aaa                                  3333
<210> 7
   <211> 3475
   <212> DNA
   <213> Homo sapiens
<400> 7
   aaagctctgg gccccaggga ggaggctcag cacagagagt ggaaaacagc agaggtgaca 60
   gagcagccgt gctcgaagcg ttcctggagc ccaagctctc ctccacaggt gaagacaggg 120
   ccagcaggag acaccatggg gcacctctca gccccacttc acagagtgcg tgtaccctgg 180
   caggggcttc tgctcacagc ctcacttcta accttctgga acccgcccac cactgcccag 240
   ctcactactg aatccatgcc attcaatgtt gcagagggga aggaggttct tctccttgtc 300
   cacaatctgc cccagcaact ttttggctac agctggtaca aaggggaaag agtggatggc 360
   aaccgtcaaa ttgtaggata tgcaatagga actcaacaag ctaccccagg gcccgcaaac 420
   agcggtcgag agacaatata ccccaatgca tccctgctga tccagaacgt cacccagaat 480
   gacacaggat tctacaccct acaagtcata aagtcagatc ttgtgaatga agaagcaact 540
   ggacagttcc atgtataccc ggagctgccc aagccctcca tctccagcaa caactccaac 600
   cctgtggagg acaaggatgc tgtggccttc acctgtgaac ctgagactca ggacacaacc 660
   tacctgtggt ggataaacaa tcagagcctc ccggtcagtc ccaggctgca gctgtccaat 720
   ggcaacagga ccctcactct actcagtgtc acaaggaatg acacaggacc ctatgagtgt 780
   gaaatacaga acccagtgag tgcgaaccgc agtgacccag tcaccttgaa tgtcacctat 840
   ggcccggaca cccccaccat ttccccttca gacacctatt accgtccagg ggcaaacctc 900
   agcctctcct gctatgcagc ctctaaccca cctgcacagt actcctggct tatcaatgga 960
   acattccagc aaagcacaca agagctcttt atccctaaca tcactgtgaa taatagtgga 1020
   tcctatacct gccacgccaa taactcagtc actggctgca acaggaccac agtcaagacg 1080
   atcatagtca ctgagctaag tccagtagta gcaaagcccc aaatcaaagc cagcaagacc 1140
   acagtcacag gagataagga ctctgtgaac ctgacctgct ccacaaatga cactggaatc 1200
   tccatccgtt ggttcttcaa aaaccagagt ctcccgtcct cggagaggat gaagctgtcc 1260
   cagggcaaca ccaccctcag cataaaccct gtcaagaggg aggatgctgg gacgtattgg 1320
   tgtgaggtct tcaacccaat cagtaagaac caaagcgacc ccatcatgct gaacgtaaac 1380
   tataatgctc taccacaaga aaatggcctc tcacctgggg ccattgctgg cattgtgatt 1440
   ggagtagtgg ccctggttgc tctgatagca gtagccctgg catgttttct gcatttcggg 1500
   aagaccggca gctcaggacc actccaatga cccacctaac aagatgaatg aagttactta 1560
   ttctaccctg aactttgaag cccagcaacc cacacaacca acttcagcct ccccatccct 1620
   aacagccaca gaaataattt attcagaagt aaaaaagcag taatgaaacc tgtcctgctc 1680
   actgcagtgc tgatgtattt caagtctctc accctcatca ctaggagatt cctttcccct 1740
   gtaggggtag aggggtgggg acagaaacaa ctttctccta ctcttccttc ctaataggca 1800
   tctccaggct gcctggtcac tgcccctctc tcagtgtcaa tagatgaaag tacattggga 1860
   gtctgtagga aacccaacct tcttgtcatt gaaatttggc aaagctgact ttgggaaaga 1920
   gggaccagaa cttcccctcc cttccccttt tcccaacctg gacttgtttt aaacttgcct 1980
   gttcagagca ctcattcctt cccaccccca gtcctgtcct atcactctaa ttcggatttg 2040
   ccatagcctt gaggttatgt ccttttccat taagtacatg tgccaggaaa caagagagag 2100
   agaaagtaaa ggcagtaatg ccttctccta tttctccaaa gccttgtgtg aactcaccaa 2160
   acacaagaaa atcaaatata taaccaatag tgaaatgcca cacctttgtc cactgtcagg 2220
   gttgtctacc tgtaggatca gggtctaagc accttggtgc ttagctagaa taccacctaa 2280
   tccttctggc aagcctgtct tcagagaacc cactagaagc aactaggaaa atcacttgcc 2340
   aaaatccaag gcaattcctg atggaaaatg caaaagcaca tatatgtttt aatatcttta 2400
   tgggctctgt tcaaggcagt gctgagaggg aggggttata gcttcaggag ggaaccagct 2460
   tctgataaac acaatctgct aggaacttgg gaaaggaatc agagagctgc ccttcagcga 2520
   ttatttaaat tattgttaaa gaatacacaa tttggggtat tgggattttt ctccttttct 2580
   ctgagacatt ccaccatttt aatttttgta actgcttatt tatgtgaaaa gggttatttt 2640
   tacttagctt agctatgtca gccaatccga ttgccttagg tgaaagaaac caccgaaatc 2700
   cctcaggtcc cttggtcagg agcctctcaa gatttttttt gtcagaggct ccaaatagaa 2760
   aataagaaaa ggttttcttc attcatggct agagctagat ttaactcagt ttctaggcac 2820
   ctcagaccaa tcatcaacta ccattctatt ccatgtttgc acctgtgcat tttctgtttg 2880
   cccccattca ctttgtcagg aaaccttggc ctctgctaag gtgtatttgg tccttgagaa 2940
   gtgggagcac cctacaggga cactatcact catgctggtg gcattgttta cagctagaaa 3000
   gctgcactgg tgctaatgcc ccttggggaa atggggctgt gaggaggagg attataactt 3060
   aggcctagcc tcttttaaca gcctctgaaa tttatctttt cttctatggg gtctataaat 3120
   gtatcttata ataaaaagga aggacaggag gaagacaggc aaatgtactt ctcacccagt 3180
   cttctacaca gatggaatct ctttggggct aagagaaagg ttttattcta tattgcttac 3240
   ctgatctcat gttaggccta agaggctttc tccaggagga ttagcttgga gttctctata 3300
   ctcaggtacc tctttcaggg ttttctaacc ctgacacgga ctgtgcatac tttccctcat 3360
   ccatgctgtg ctgtgttatt taatttttcc tggctaagat catgtctgaa ttatgtatga 3420
   aaattattct atgtttttat aataaaaata atatatcaga catcgaaaaa aaaaa 3475
<210> 8
   <211> 3528
   <212> DNA
   <213> Homo sapiens
<400> 8
   aaagctctgg gccccaggga ggaggctcag cacagagagt ggaaaacagc agaggtgaca      60
   gagcagccgt gctcgaagcg ttcctggagc ccaagctctc ctccacaggt gaagacaggg     120
   ccagcaggag acaccatggg gcacctctca gccccacttc acagagtgcg tgtaccctgg     180
   caggggcttc tgctcacagc ctcacttcta accttctgga acccgcccac cactgcccag     240
   ctcactactg aatccatgcc attcaatgtt gcagagggga aggaggttct tctccttgtc     300
   cacaatctgc cccagcaact ttttggctac agctggtaca aaggggaaag agtggatggc     360
   aaccgtcaaa ttgtaggata tgcaatagga actcaacaag ctaccccagg gcccgcaaac     420
   agcggtcgag agacaatata ccccaatgca tccctgctga tccagaacgt cacccagaat     480
   gacacaggat tctacaccct acaagtcata aagtcagatc ttgtgaatga agaagcaact     540
   ggacagttcc atgtataccc ggagctgccc aagccctcca tctccagcaa caactccaac     600
   cctgtggagg acaaggatgc tgtggccttc acctgtgaac ctgagactca ggacacaacc     660
   tacctgtggt ggataaacaa tcagagcctc ccggtcagtc ccaggctgca gctgtccaat     720
   ggcaacagga ccctcactct actcagtgtc acaaggaatg acacaggacc ctatgagtgt     780
   gaaatacaga acccagtgag tgcgaaccgc agtgacccag tcaccttgaa tgtcacctat     840
   ggcccggaca cccccaccat ttccccttca gacacctatt accgtccagg ggcaaacctc     900
   agcctctcct gctatgcagc ctctaaccca cctgcacagt actcctggct tatcaatgga     960
   acattccagc aaagcacaca agagctcttt atccctaaca tcactgtgaa taatagtgga    1020
   tcctatacct gccacgccaa taactcagtc actggctgca acaggaccac agtcaagacg    1080
   atcatagtca ctgagctaag tccagtagta gcaaagcccc aaatcaaagc cagcaagacc    1140
   acagtcacag gagataagga ctctgtgaac ctgacctgct ccacaaatga cactggaatc    1200
   tccatccgtt ggttcttcaa aaaccagagt ctcccgtcct cggagaggat gaagctgtcc    1260
   cagggcaaca ccaccctcag cataaaccct gtcaagaggg aggatgctgg gacgtattgg    1320
   tgtgaggtct tcaacccaat cagtaagaac caaagcgacc ccatcatgct gaacgtaaac    1380
   tataatgctc taccacaaga aaatggcctc tcacctgggg ccattgctgg cattgtgatt    1440
   ggagtagtgg ccctggttgc tctgatagca gtagccctgg catgttttct gcatttcggg    1500
   aagaccggca gggcaagcga ccagcgtgat ctcacagagc acaaaccctc agtctccaac    1560
   cacactcagg accactccaa tgacccacct aacaagatga atgaagttac ttattctacc    1620
   ctgaactttg aagcccagca acccacacaa ccaacttcag cctccccatc cctaacagcc    1680
   acagaaataa tttattcaga agtaaaaaag cagtaatgaa acctgtcctg ctcactgcag    1740
   tgctgatgta tttcaagtct ctcaccctca tcactaggag attcctttcc cctgtagggg    1800
   tagaggggtg gggacagaaa caactttctc ctactcttcc ttcctaatag gcatctccag    1860
   gctgcctggt cactgcccct ctctcagtgt caatagatga aagtacattg ggagtctgta 1920
   ggaaacccaa ccttcttgtc attgaaattt ggcaaagctg actttgggaa agagggacca 1980
   gaacttcccc tcccttcccc ttttcccaac ctggacttgt tttaaacttg cctgttcaga 2040
   gcactcattc cttcccaccc ccagtcctgt cctatcactc taattcggat ttgccatagc 2100
   cttgaggtta tgtccttttc cattaagtac atgtgccagg aaacaagaga gagagaaagt 2160
   aaaggcagta atgccttctc ctatttctcc aaagccttgt gtgaactcac caaacacaag 2220
   aaaatcaaat atataaccaa tagtgaaatg ccacaccttt gtccactgtc agggttgtct 2280
   acctgtagga tcagggtcta agcaccttgg tgcttagcta gaataccacc taatccttct 2340
   ggcaagcctg tcttcagaga acccactaga agcaactagg aaaatcactt gccaaaatcc 2400
   aaggcaattc ctgatggaaa atgcaaaagc acatatatgt tttaatatct ttatgggctc 2460
   tgttcaaggc agtgctgaga gggaggggtt atagcttcag gagggaacca gcttctgata 2520
   aacacaatct gctaggaact tgggaaagga atcagagagc tgcccttcag cgattattta 2580
   aattattgtt aaagaataca caatttgggg tattgggatt tttctccttt tctctgagac 2640
   attccaccat tttaattttt gtaactgctt atttatgtga aaagggttat ttttacttag 2700
   cttagctatg tcagccaatc cgattgcctt aggtgaaaga aaccaccgaa atccctcagg 2760
   tcccttggtc aggagcctct caagattttt tttgtcagag gctccaaata gaaaataaga 2820
   aaaggttttc ttcattcatg gctagagcta gatttaactc agtttctagg cacctcagac 2880
   caatcatcaa ctaccattct attccatgtt tgcacctgtg cattttctgt ttgcccccat 2940
   tcactttgtc aggaaacctt ggcctctgct aaggtgtatt tggtccttga gaagtgggag 3000
   caccctacag ggacactatc actcatgctg gtggcattgt ttacagctag aaagctgcac 3060
   tggtgctaat gccccttggg gaaatggggc tgtgaggagg aggattataa cttaggccta 3120
   gcctctttta acagcctctg aaatttatct tttcttctat ggggtctata aatgtatctt 3180
   ataataaaaa ggaaggacag gaggaagaca ggcaaatgta cttctcaccc agtcttctac 3240
   acagatggaa tctctttggg gctaagagaa aggttttatt ctatattgct tacctgatct 3300
   catgttaggc ctaagaggct ttctccagga ggattagctt ggagttctct atactcaggt 3360
   acctctttca gggttttcta accctgacac ggactgtgca tactttccct catccatgct 3420
   gtgctgtgtt atttaatttt tcctggctaa gatcatgtct gaattatgta tgaaaattat 3480
   tctatgtttt tataataaaa ataatatatc agacatcgaa aaaaaaaa 3528
<210> 9
   <211> 493
   <212> PRT
   <213> Homo sapiens
<400> 9
   Gly Arg Gly Asp Arg Ala Ala Val Leu Glu Ala Phe Leu Glu Pro Lys
   1               5                   10                  15
   Leu Ser Ser Thr Gly Glu Asp Arg Ala Ser Arg Arg His His Gly Ala
               20                  25                  30
   Pro Leu Ser Pro Thr Ser Gln Ser Ala Cys Thr Leu Ala Gly Leu Leu
   35 40 45
   Leu Thr Ala Ser Leu Leu Thr Phe Trp Asn Pro Pro Thr Thr Ala Gln
   50 55 60
   Leu Thr Thr Glu Ser Met Pro Phe Asn Val Ala Glu Gly Lys Glu Val
   65 70 75 80
   Leu Leu Leu Val His Asn Leu Pro Gln Gln Leu Phe Gly Tyr Ser Trp
   85 90 95
   Tyr Lys Gly Glu Arg Val Asp Gly Asn Arg Gln Ile Val Gly Tyr Ala
   100 105 110
   Ile Gly Thr Gln Gln Ala Thr Pro Gly Pro Ala Asn Ser Gly Arg Glu
   115 120 125
   Thr Ile Tyr Pro Asn Ala Ser Leu Leu Ile Gln Asn Val Thr Gln Asn
   130 135 140
   Asp Thr Gly Phe Tyr Thr Leu Gln Val Ile Lys Ser Asp Leu Val Asn
   145 150 155 160
   Glu Glu Ala Thr Gly Gln Phe His Val Tyr Pro Glu Leu Pro Lys Pro
   165 170 175
   Ser Ile Ser Ser Asn Asn Ser Asn Pro Val Glu Asp Lys Asp Ala Val
   180 185 190
   Ala Phe Thr Cys Glu Pro Glu Thr Gln Asp Thr Thr Tyr Leu Trp Trp
   195 200 205
   Ile Asn Asn Gln Ser Leu Pro Val Ser Pro Arg Leu Gln Leu Ser Asn
   210 215 220
   Gly Asn Arg Thr Leu Thr Leu Leu Ser Val Thr Arg Asn Asp Thr Gly
   225 230 235 240
   Pro Tyr Glu Cys Glu Ile Gln Asn Pro Val Ser Ala Asn Arg Ser Asp
   245 250 255
   Pro Val Thr Leu Asn Val Thr Tyr Gly Pro Asp Thr Pro Thr Ile Ser
   260 265 270
   Pro Ser Asp Thr Tyr Tyr Arg Pro Gly Ala Asn Leu Ser Leu Ser Cys
   275 280 285
   Tyr Ala Ala Ser Asn Pro Pro Ala Gln Tyr Ser Trp Leu Ile Asn Gly
   290 295 300
   Thr Phe Gln Gln Ser Thr Gln Glu Leu Phe Ile Pro Asn Ile Thr Val
   305 310 315 320
   Asn Asn Ser Gly Ser Tyr Thr Cys His Ala Asn Asn Ser Val Thr Gly
   325 330 335
   Cys Asn Arg Thr Thr Val Lys Thr Ile Ile Val Thr Glu Leu Ser Pro
               340                 345                 350
   Val Val Ala Lys Pro Gln Ile Lys Ala Ser Lys Thr Thr Val Thr Gly
           355                 360                 365
   Asp Lys Asp Ser Val Asp Leu Thr Cys Ser Thr Asn Asp Thr Gly Ile
       370                 375                 380
   Ser Ile Arg Trp Phe Phe Lys Asn Gln Ser Leu Pro Ser Ser Glu Arg
   385                 390                 395                 400
   Met Lys Leu Ser Gln Gly Asn Thr Thr Leu Ser Ile Asn Pro Val Lys
                   405                 410                 415
   Arg Glu Asp Ala Gly Thr Tyr Trp Cys Glu Val Phe Asn Pro Ile Ser
               420                 425                 430
   Lys Asn Gln Ser Asp Pro Ile Met Leu Asn Val Asn Tyr Asn Ala Leu
           435                 440                 445
   Pro Gln Glu Asn Gly Leu Ser Pro Gly Ala Ile Ala Gly Ile Val Ile
       450                 455                 460
   Gly Val Val Ala Leu Val Ala Leu Ile Ala Val Ala Leu Ala Cys Phe
   465                 470                 475                 480
   Leu His Phe Gly Lys Thr Gly Ser Ser Gly Pro Leu Gln
                   485                 490
<210> 10
   <211> 468
   <212> PRT
   <213> Homo sapiens
<400> 10
   Met Gly His Leu Ser Ala Pro Leu His Arg Val Arg Val Pro Trp Gln
   1               5                   10                  15
   Gly Leu Leu Leu Thr Ala Ser Leu Leu Thr Phe Trp Asn Pro Pro Thr
               20                  25                  30
   Thr Ala Gln Leu Thr Thr Glu Ser Met Pro Phe Asn Val Ala Glu Gly
           35                  40                  45
   Lys Glu Val Leu Leu Leu Val His Asn Leu Pro Gln Gln Leu Phe Gly
       50                  55                  60
   Tyr Ser Trp Tyr Lys Gly Glu Arg Val Asp Gly Asn Arg Gln Ile Val
   65                  70                  75                  80
   Gly Tyr Ala Ile Gly Thr Gln Gln Ala Thr Pro Gly Pro Ala Asn Ser
                   85                  90                  95
   Gly Arg Glu Thr Ile Tyr Pro Asn Ala Ser Leu Leu Ile Gln Asn Val
               100                 105                 110
   Thr Gln Asn Asp Thr Gly Phe Tyr Thr Leu Gln Val Ile Lys Ser Asp
           115                 120                 125
   Leu Val Asn Glu Glu Ala Thr Gly Gln Phe His Val Tyr Pro Glu Leu
       130                 135                 140
   Pro Lys Pro Ser Ile Ser Ser Asn Asn Ser Asn Pro Val Glu Asp Lys
   145                 150                 155                 160
   Asp Ala Val Ala Phe Thr Cys Glu Pro Glu Thr Gln Asp Thr Thr Tyr
                   165                 170                 175
   Leu Trp Trp Ile Asn Asn Gln Ser Leu Pro Val Ser Pro Arg Leu Gln
               180                 185                 190
   Leu Ser Asn Gly Asn Arg Thr Leu Thr Leu Leu Ser Val Thr Arg Asn
           195                 200                 205
   Asp Thr Gly Pro Tyr Glu Cys Glu Ile Gln Asn Pro Val Ser Ala Asn
       210                 215                 220
   Arg Ser Asp Pro Val Thr Leu Asn Val Thr Tyr Gly Pro Asp Thr Pro
   225                 230                 235                 240
   Thr Ile Ser Pro Ser Asp Thr Tyr Tyr Arg Pro Gly Ala Asn Leu Ser
                   245                 250                 255
   Leu Ser Cys Tyr Ala Ala Ser Asn Pro Pro Ala Gln Tyr Ser Trp Leu
               260                 265                 270
   Ile Asn Gly Thr Phe Gln Gln Ser Thr Gln Glu Leu Phe Ile Pro Asn
           275                 280                 285
   Ile Thr Val Asn Asn Ser Gly Ser Tyr Thr Cys His Ala Asn Asn Ser
       290                 295                 300
   Val Thr Gly Cys Asn Arg Thr Thr Val Lys Thr Ile Ile Val Thr Glu
   305                 310                 315                 320
   Leu Ser Pro Val Val Ala Lys Pro Gln Ile Lys Ala Ser Lys Thr Thr
                   325                 330                 335
   Val Thr Gly Asp Lys Asp Ser Val Asn Leu Thr Cys Ser Thr Asn Asp
               340                 345                 350
   Thr Gly Ile Ser Ile Arg Trp Phe Phe Lys Asn Gln Ser Leu Pro Ser
           355                 360                 365
   Ser Glu Arg Met Lys Leu Ser Gln Gly Asn Thr Thr Leu Ser Ile Asn
       370                 375                 380
   Pro Val Lys Arg Glu Asp Ala Gly Thr Tyr Trp Cys Glu Val Phe Asn
   385                 390                 395                 400
   Pro Ile Ser Lys Asn Gln Ser Asp Pro Ile Met Leu Asn Val Asn Tyr
   405 410 415
   Asn Ala Leu Pro Gln Glu Asn Gly Leu Ser Pro Gly Ala Ile Ala Gly
   420 425 430
   Ile Val Ile Gly Val Val Ala Leu Val Ala Leu Ile Ala Val Ala Leu
   435 440 445
   Ala Cys Phe Leu His Phe Gly Lys Thr Gly Arg Thr Thr Pro Met Thr
   450 455 460
   His Leu Thr Arg
   465
<210> 11
   <211> 368
   <212> PRT
   <213> Homo sapiens
<400> 11
   Met Gly His Leu Ser Ala Pro Leu His Arg Val Arg Val Pro Trp Gln
   1 5 10 15
   Gly Leu Leu Leu Thr Ala Ser Leu Leu Thr Phe Trp Asn Pro Pro Thr
   20 25 30
   Thr Ala Gln Leu Thr Thr Glu Ser Met Pro Phe Asn Val Ala Glu Gly
   35 40 45
   Lys Glu Val Leu Leu Leu Val His Asn Leu Pro Gln Gln Leu Phe Gly
   50 55 60
   Tyr Ser Trp Tyr Lys Gly Glu Arg Val Asp Gly Asn Arg Gln Ile Val
   65 70 75 80
   Gly Tyr Ala Ile Gly Thr Gln Gln Ala Thr Pro Gly Pro Ala Asn Ser
   85 90 95
   Gly Arg Glu Thr Ile Tyr Pro Asn Ala Ser Leu Leu Ile Gln Asn Val
   100 105 110
   Thr Gln Asn Asp Thr Gly Phe Tyr Thr Leu Gln Val Ile Lys Ser Asp
   115 120 125
   Leu Val Asn Glu Glu Ala Thr Gly Gln Phe His Val Tyr Pro Glu Leu
   130 135 140
   Pro Lys Pro Ser Ile Ser Ser Asn Asn Ser Asn Pro Val Glu Asp Lys
   145 150 155 160
   Asp Ala Val Ala Phe Thr Cys Glu Pro Glu Thr Gln Asp Thr Thr Tyr
   165 170 175
   Leu Trp Trp Ile Asn Asn Gln Ser Leu Pro Val Ser Pro Arg Leu Gln
   180 185 190
   Leu Ser Asn Gly Asn Arg Thr Leu Thr Leu Leu Ser Val Thr Arg Asn
           195                 200                 205
   Asp Thr Gly Pro Tyr Glu Cys Glu Ile Gln Asn Pro Val Ser Ala Asn
       210                 215                 220
   Arg Ser Asp Pro Val Thr Leu Asn Val Thr Tyr Gly Pro Asp Thr Pro
   225                 230                 235                 240
   Thr Ile Ser Pro Ser Asp Thr Tyr Tyr Arg Pro Gly Ala Asn Leu Ser
                   245                 250                 255
   Leu Ser Cys Tyr Ala Ala Ser Asn Pro Pro Ala Gln Tyr Ser Trp Leu
               260                 265                 270
   Ile Asn Gly Thr Phe Gln Gln Ser Thr Gln Glu Leu Phe Ile Pro Asn
           275                 280                 285
   Ile Thr Val Asn Asn Ser Gly Ser Tyr Thr Cys His Ala Asn Asn Ser
       290                 295                 300
   Val Thr Gly Cys Asn Arg Thr Thr Val Lys Thr Ile Ile Val Thr Asp
   305                 310                 315                 320
   Asn Ala Leu Pro Gln Glu Asn Gly Leu Ser Pro Gly Ala Ile Ala Gly
                   325                 330                 335
   Ile Val Ile Gly Val Val Ala Leu Val Ala Leu Ile Ala Val Ala Leu
               340                 345                 350
   Ala Cys Phe Leu His Phe Gly Lys Thr Gly Ser Ser Gly Pro Leu Gln
           355                 360                 365
<210> 12
   <211> 430
   <212> PRT
   <213> Homo sapiens
<400> 12
   Met Gly His Leu Ser Ala Pro Leu His Arg Val Arg Val Pro Trp Gln
   1               5                   10                  15
   Gly Leu Leu Leu Thr Ala Ser Leu Leu Thr Phe Trp Asn Pro Pro Thr
               20                  25                  30
   Thr Ala Gln Leu Thr Thr Glu Ser Met Pro Phe Asn Val Ala Glu Gly
           35                  40                  45
   Lys Glu Val Leu Leu Leu Val His Asn Leu Pro Gln Gln Leu Phe Gly
       50                  55                  60
   Tyr Ser Trp Tyr Lys Gly Glu Arg Val Asp Gly Asn Arg Gln Ile Val
   65                  70                  75                  80
   Gly Tyr Ala Ile Gly Thr Gln Gln Ala Thr Pro Gly Pro Ala Asn Ser
                   85                  90                  95
   Gly Arg Glu Thr Ile Tyr Pro Asn Ala Ser Leu Leu Ile Gln Asn Val
               100                 105                 110
   Thr Gln Asn Asp Thr Gly Phe Tyr Thr Leu Gln Val Ile Lys Ser Asp
           115                 120                 125
   Leu Val Asn Glu Glu Ala Thr Gly Gln Phe His Val Tyr Pro Glu Leu
       130                 135                 140
   Pro Lys Pro Ser Ile Ser Ser Asn Asn Ser Asn Pro Val Glu Asp Lys
   145                 150                 155                 160
   Asp Ala Val Ala Phe Thr Cys Glu Pro Glu Thr Gln Asp Thr Thr Tyr
                   165                 170                 175
   Leu Trp Trp Ile Asn Asn Gln Ser Leu Pro Val Ser Pro Arg Leu Gln
               180                 185                 190
   Leu Ser Asn Gly Asn Arg Thr Leu Thr Leu Leu Ser Val Thr Arg Asn
           195                 200                 205
   Asp Thr Gly Pro Tyr Glu Cys Glu Ile Gln Asn Pro Val Ser Ala Asn
       210                 215                 220
   Arg Ser Asp Pro Val Thr Leu Asn Val Thr Tyr Gly Pro Asp Thr Pro
   225                 230                 235                 240
   Thr Ile Ser Pro Ser Asp Thr Tyr Tyr Arg Pro Gly Ala Asn Leu Ser
                   245                 250                 255
   Leu Ser Cys Tyr Ala Ala Ser Asn Pro Pro Ala Gln Tyr Ser Trp Leu
               260                 265                 270
   Ile Asn Gly Thr Phe Gln Gln Ser Thr Gln Glu Leu Phe Ile Pro Asn
           275                 280                 285
   Ile Thr Val Asn Asn Ser Gly Ser Tyr Thr Cys His Ala Asn Asn Ser
       290                 295                 300
   Val Thr Gly Cys Asn Arg Thr Thr Val Lys Thr Ile Ile Val Thr Asp
   305                 310                 315                 320
   Asn Ala Leu Pro Gln Glu Asn Gly Leu Ser Pro Gly Ala Ile Ala Gly
                   325                 330                 335
   Ile Val Ile Gly Val Val Ala Leu Val Ala Leu Ile Ala Val Ala Leu
               340                 345                 350
   Ala Cys Phe Leu His Phe Gly Lys Thr Gly Arg Ala Ser Asp Gln Arg
           355                 360                 365
   Asp Leu Thr Glu His Lys Pro Ser Val Ser Asn His Thr Gln Asp His
       370                 375                 380
   Ser Asn Asp Pro Pro Asn Lys Met Asn Glu Val Thr Tyr Ser Thr Leu
   385                 390                 395                 400
   Asn Phe Glu Ala Gln Gln Pro Thr Gln Pro Thr Ser Ala Ser Pro Ser
                   405                 410                 415
   Leu Thr Ala Thr Glu Ile Ile Tyr Ser Glu Val Lys Lys Gln
               420                 425                 430
<210> 13
   <211> 461
   <212> PRT
   <213> Homo sapiens
<400> 13
   Met Gly His Leu Ser Ala Pro Leu His Arg Val Arg Val Pro Trp Gln
   1               5                   10                  15
   Gly Leu Leu Leu Thr Ala Ser Leu Leu Thr Phe Trp Asn Pro Pro Thr
               20                  25                  30
   Thr Ala Gln Leu Thr Thr Glu Ser Met Pro Phe Asn Val Ala Glu Gly
           35                  40                  45
   Lys Glu Val Leu Leu Leu Val His Asn Leu Pro Gln Gln Leu Phe Gly
       50                  55                  60
   Tyr Ser Trp Tyr Lys Gly Glu Arg Val Asp Gly Asn Arg Gln Ile Val
   65                  70                  75                  80
   Gly Tyr Ala Ile Gly Thr Gln Gln Ala Thr Pro Gly Pro Ala Asn Ser
                   85                  90                  95
   Gly Arg Glu Thr Ile Tyr Pro Asn Ala Ser Leu Leu Ile Gln Asn Val
               100                 105                 110
   Thr Gln Asn Asp Thr Gly Phe Tyr Thr Leu Gln Val Ile Lys Ser Asp
           115                 120                 125
   Leu Val Asn Glu Glu Ala Thr Gly Gln Phe His Val Tyr Pro Glu Leu
       130                 135                 140
   Pro Lys Pro Ser Ile Ser Ser Asn Asn Ser Asn Pro Val Glu Asp Lys
   145                 150                 155                 160
   Asp Ala Val Ala Phe Thr Cys Glu Pro Glu Thr Gln Asp Thr Thr Tyr
                   165                 170                 175
   Leu Trp Trp Ile Asn Asn Gln Ser Leu Pro Val Ser Pro Arg Leu Gln
               180                 185                 190
   Leu Ser Asn Gly Asn Arg Thr Leu Thr Leu Leu Ser Val Thr Arg Asn
           195                 200                 205
   Asp Thr Gly Pro Tyr Glu Cys Glu Ile Gln Asn Pro Val Ser Ala Asn
       210                 215                 220
   Arg Ser Asp Pro Val Thr Leu Asn Val Thr Tyr Gly Pro Asp Thr Pro
   225                 230                 235                 240
   Thr Ile Ser Pro Ser Asp Thr Tyr Tyr Arg Pro Gly Ala Asn Leu Ser
                   245                 250                 255
   Leu Ser Cys Tyr Ala Ala Ser Asn Pro Pro Ala Gln Tyr Ser Trp Leu
               260                 265                 270
   Ile Asn Gly Thr Phe Gln Gln Ser Thr Gln Glu Leu Phe Ile Pro Asn
           275                 280                 285
   Ile Thr Val Asn Asn Ser Gly Ser Tyr Thr Cys His Ala Asn Asn Ser
       290                 295                 300
   Val Thr Gly Cys Asn Arg Thr Thr Val Lys Thr Ile Ile Val Thr Glu
   305                 310                 315                 320
   Arg Gln Asn Leu Thr Met Leu Pro Arg Leu Asp Ser Asn Ser Trp Ala
                   325                 330                 335
   Gln Ala Ile Leu Pro Ser Val Ser Gln Ser Ala Glu Ile Thr Asp Asn
               340                 345                 350
   Ala Leu Pro Gln Glu Asn Gly Leu Ser Pro Gly Ala Ile Ala Gly Ile
           355                 360                 365
   Val Ile Gly Val Val Ala Leu Val Ala Leu Ile Ala Val Ala Leu Ala
       370                 375                 380
   Cys Phe Leu His Phe Gly Lys Thr Gly Arg Ala Ser Asp Gln Arg Asp
   385                 390                 395                 400
   Leu Thr Glu His Lys Pro Ser Val Ser Asn His Thr Gln Asp His Ser
                   405                 410                 415
   Asn Asp Pro Pro Asn Lys Met Asn Glu Val Thr Tyr Ser Thr Leu Asn
               420                 425                 430
   Phe Glu Ala Gln Gln Pro Thr Gln Pro Thr Ser Ala Ser Pro Ser Leu
           435                 440                 445
   Thr Ala Thr Glu Ile Ile Tyr Ser Glu Val Lys Lys Gln
       450                 455                 460
<210> 14
   <211> 526
   <212> PRT
   <213> Homo sapiens
<400> 14
   Met Gly His Leu Ser Ala Pro Leu His Arg Val Arg Val Pro Trp Gln
   1               5                   10                  15
   Gly Leu Leu Leu Thr Ala Ser Leu Leu Thr Phe Trp Asn Pro Pro Thr
               20                  25                  30
   Thr Ala Gln Leu Thr Thr Glu Ser Met Pro Phe Asn Val Ala Glu Gly
           35                  40                  45
   Lys Glu Val Leu Leu Leu Val His Asn Leu Pro Gln Gln Leu Phe Gly
       50                  55                  60
   Tyr Ser Trp Tyr Lys Gly Glu Arg Val Asp Gly Asn Arg Gln Ile Val
   65                  70                  75                  80
   Gly Tyr Ala Ile Gly Thr Gln Gln Ala Thr Pro Gly Pro Ala Asn Ser
                   85                  90                  95
   Gly Arg Glu Thr Ile Tyr Pro Asn Ala Ser Leu Leu Ile Gln Asn Val
               100                 105                 110
   Thr Gln Asn Asp Thr Gly Phe Tyr Thr Leu Gln Val Ile Lys Ser Asp
           115                 120                 125
   Leu Val Asn Glu Glu Ala Thr Gly Gln Phe His Val Tyr Pro Glu Leu
       130                 135                 140
   Pro Lys Pro Ser Ile Ser Ser Asn Asn Ser Asn Pro Val Glu Asp Lys
   145                 150                 155                 160
   Asp Ala Val Ala Phe Thr Cys Glu Pro Glu Thr Gln Asp Thr Thr Tyr
                   165                 170                 175
   Leu Trp Trp Ile Asn Asn Gln Ser Leu Pro Val Ser Pro Arg Leu Gln
               180                 185                 190
   Leu Ser Asn Gly Asn Arg Thr Leu Thr Leu Leu Ser Val Thr Arg Asn
           195                 200                 205
   Asp Thr Gly Pro Tyr Glu Cys Glu Ile Gln Asn Pro Val Ser Ala Asn
       210                 215                 220
   Arg Ser Asp Pro Val Thr Leu Asn Val Thr Tyr Gly Pro Asp Thr Pro
   225                 230                 235                 240
   Thr Ile Ser Pro Ser Asp Thr Tyr Tyr Arg Pro Gly Ala Asn Leu Ser
                   245                 250                 255
   Leu Ser Cys Tyr Ala Ala Ser Asn Pro Pro Ala Gln Tyr Ser Trp Leu
               260                 265                 270
   Ile Asn Gly Thr Phe Gln Gln Ser Thr Gln Glu Leu Phe Ile Pro Asn
           275                 280                 285
   Ile Thr Val Asn Asn Ser Gly Ser Tyr Thr Cys His Ala Asn Asn Ser
       290                 295                 300
   Val Thr Gly Cys Asn Arg Thr Thr Val Lys Thr Ile Ile Val Thr Glu
   305                 310                 315                 320
   Leu Ser Pro Val Val Ala Lys Pro Gln Ile Lys Ala Ser Lys Thr Thr
                   325                 330                 335
   Val Thr Gly Asp Lys Asp Ser Val Asn Leu Thr Cys Ser Thr Asn Asp
               340                 345                 350
   Thr Gly Ile Ser Ile Arg Trp Phe Phe Lys Asn Gln Ser Leu Pro Ser
           355                 360                 365
   Ser Glu Arg Met Lys Leu Ser Gln Gly Asn Thr Thr Leu Ser Ile Asn
       370                 375                 380
   Pro Val Lys Arg Glu Asp Ala Gly Thr Tyr Trp Cys Glu Val Phe Asn
   385                 390                 395                 400
   Pro Ile Ser Lys Asn Gln Ser Asp Pro Ile Met Leu Asn Val Asn Tyr
                   405                 410                 415
   Asn Ala Leu Pro Gln Glu Asn Gly Leu Ser Pro Gly Ala Ile Ala Gly
               420                 425                 430
   Ile Val Ile Gly Val Val Ala Leu Val Ala Leu Ile Ala Val Ala Leu
           435                 440                 445
   Ala Cys Phe Leu His Phe Gly Lys Thr Gly Arg Ala Ser Asp Gln Arg
       450                 455                 460
   Asp Leu Thr Glu His Lys Pro Ser Val Ser Asn His Thr Gln Asp His
   465                 470                 475                 480
   Ser Asn Asp Pro Pro Asn Lys Met Asn Glu Val Thr Tyr Ser Thr Leu
                   485                 490                 495
   Asn Phe Glu Ala Gln Gln Pro Thr Gln Pro Thr Ser Ala Ser Pro Ser
               500                 505                 510
   Leu Thr Ala Thr Glu Ile Ile Tyr Ser Glu Val Lys Lys Gln
           515                 520                 525

## Description

### RELATED APPLICATION/S

This application claims the benefit of priority under 35 USC 119(e) of U.S. Provisional Patent Application No. 61/227,130 filed July 21, 2009, the contents of which are incorporated herein by reference in their entirety.

### FIELD AND BACKGROUND OF THE INVENTION

The present invention, in some embodiments thereof, relates to methods for diagnosing cancer, specifically melanoma.

Cancer is a class of diseases in which a group of cells display *uncontrolled growth* (division beyond the normal limits), *invasion* (intrusion on and destruction of adjacent tissues), and sometimes *metastasis.* Cancer affects people at all ages with the risk for most types increasing with age. Cancer caused about 13 % of all human deaths in 2007 (7.6 million).

There is a need for a rapid, economical and accurate diagnostic test for cancer.

The human Carcinoembryonic Ag (CEA) protein family encompasses several forms of proteins with different biochemical features. All CEA family genes have been classified into two major subfamilies, the CEA cell adhesion molecule (CEACAM) and the pregnancy-specific glycoprotein subgroups. The CEACAM proteins, which are part of the larger Ig superfamily, include CEACAM1, -3, -4, -5, - 6, -7, and -8. They share a common basic structure of sequentially ordered different Ig-like domain(s) with considerable degree of homology. CEACAM5 is GPI-linked to cell surface, but it also appears in a soluble form in the peripheral blood where it is more recognized as the tumor marker CEA used to monitor colorectal cancer patients. CEACAM 1 is a transmembrane protein that can be detected on some immune cells as well as on epithelial cells [Hammarström S (1999). Semin Cancer Biol 9: 67-81]. A striking association was observed between the presence of cell-bound CEACAM1 on primary cutaneous melanoma lesions and the development of metastatic disease with poor prognosis [Thies A et al. (2002) J Clin Oncol 20: 2530-2536]. The prognostic strength of melanoma associated CEACAM 1 was similar or even superior to the widely accepted Breslow score [Thies et al. Supra]. Remarkably, a similar association was observed in lung adenocarcinoma specifically [Laack et al (2002) J Clin Oncol 20: 4279-4284] but also generally in non small cell lung cancers [Sienel et al (2003) Clin Cancer Res 9: 2260-2266].

Interestingly, the presence of human soluble CEACAM1 protein has been observed in the serum of healthy donors [Dráberová et al (2000) Immunology 101: 279-287; Kondo et al (2001). J Gastroenterol 36: 470-475; Svenberg et al. (1979) Clin Exp Immunol 36:317-325] and was found elevated in the sera of patients with biliary diseases including obstructive jaundice, primary biliary cirrhosis, autoimmune hepatitis and cholangiocarcinoma. Furthermore, it has been recently shown that serum CEACAM1 level is increased in some pancreatic adenocarcinoma patients [Simeone et al (2007) Pancreas 34: 436-443], presenting evidence for the potential role of soluble CEACAM1 as a tumor marker.. Normally, circulating lymphocytes do not express CEACAM1 [Moller et al (1996) Int J Cancer 65: 740-745; Kammerer et al (1998) Eur J Immunol 28: 3664-3674], as it is upregulated on lymphocytes mainly following activation.

U.S. Application 20070071758 relates to methods for diagnosing cancer in vitro and in vivo using a CEACAM1 binding agent conjugated to a detectable moiety.

U.S. Application 20080108140 regards CEACAM1 as a biomarker for congenital CMV infection.

U.S. Application 20090181403 regards the utilization of CEACAM1 as a biomarker for cancer. In particular this application regards the detection and measurement of soluble CEACAM1 levels for the detection and diagnosis of melanoma.

US 2007/110668 describes the identification of a Class I MHC-Independent inhibitory mechanism of human NK cytotoxicity in a family of TAP2-deficient patients. It was found that although TAP2-deficiency leads to a reduction in Class I MHC expression, NK-mediated cytotoxicity is still inhibited by homophilic CEACAM1 interactions.

Gray-Owen et al. summarizes in Nature Reviews Immunology, vol. 6, Nr. 6, p. 433-446, a review article, the role of CEACAM proteins in infection, inflammation and cancer. It is reported that CEACAM 1 is involved in immunity, which acts as a regulatory co-receptor for lymphoid and myelod cell types.

Kammerer et al. describe in Journal of Pathology 200411, vol. 204, no. 3, p. 258-267 an investigation of CEACAM1 expression in normal kidney and renal cell carcinomas (RCC), and found that CEACAM1 expression was completely downregulated in all RCC samples, but could be re-induced by stimulation with interferon-y (IFN-γ). The authors also characterized TILs from five RCCs and found that CEACAM1 expression on NK and T cells freshly isolated from the tumor tissue was either undetectable (NK cells) or low (T cells), which is considered characteristic for unstimulated cells.

### SUMMARY OF THE INVENTION

According to an aspect of some embodiments of the present invention there is provided a method of diagnosing melanoma, the method comprising determining a level of CEACAM1 on isolated peripheral blood lymphocytes (PBLs) of a subject in need thereof, wherein an upregulation of the level of CEACAM1 above a predetermined threshold is indicative of melanoma in the subject.

According to some embodiments of the invention, the PBLs comprise T cells.

According to some embodiments of the invention, the PBLs comprise NK cells.

According to some embodiments of the invention, the determining a level of CEACAM1 on isolated peripheral blood lymphocytes (PBLs) is effected by FACS. The method may further comprise informing the subject on the presence or absence of the melanoma or stage thereof.

According to some embodiments of the invention, the method further comprising validating the diagnosis or prognosis using a method selected from the group consisting of surgical biopsy, imaging, pathology and molecular testing..

The determining is effected ex vivo.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

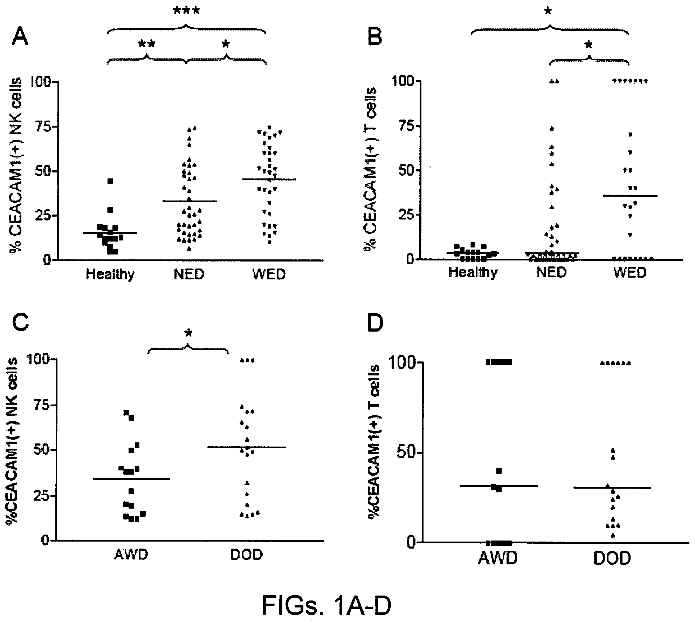

FIGs. 1A-D are graphs illustrating high expression of CEACAM1 on circulating lymphocytes among melanoma patients. A scatter distribution of CEACAM1 expression profile on circulating NK cells is shown in Figure 1a or T cells in Figure 1b. There are three main groups of samples, healthy donors (black squares), patients with no evidence of disease (NED, black upright triangles) and patients with evidence of disease (WED, black inverse triangles). Each individual shape represents a single sample from the same group. Y axis denotes the percent of CEACAM1-positive circulating lymphocytes. (Figures 1c-d) WED patients were further categorized into patients that died of disease (DOD) during follow up and patients that remained alive with disease (AWD). Figure shows CEACAM1 scatter distribution on circulating NK cells (Figure 1c) or T cells (Figure 1d) in these subgroups. Horizontal lines indicate the median value of the group. Non parametric two sided t-test was used to compare between different groups, as indicated in each plot. * denotes P value < 0.05, ** denotes P value < 0.01 and *** denotes P value < 0.001.

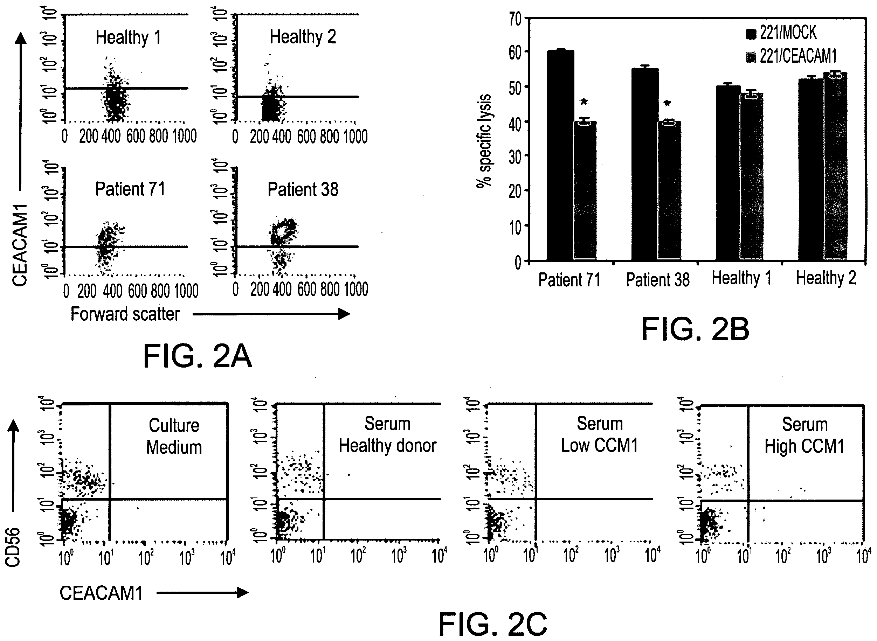

FIGs. 2A-C are graphs showing enhanced CEACAM1 functional expression that inhibits NK killing activity. Figure 2a - Plots show the CEACAM1 expression profile on gated peripheral blood NK cells. Samples were derived either from healthy donors or melanoma patients, as indicated in the figure. Figure 2b - Peripheral blood lymphocytes were tested for natural killing activity against NK-sensitive 221 cells. Target cells were either mock transfected (221/Mock - black bars) or stable transfected with CEACAM1 cDNA (221/CEACAM1 - gray bars). Effector-to-target ratio was 50-to-1. Y-axis denotes the percent of specific lysis of target cells. Figure shows a representative experiment out of 3 performed. * denotes P value < 0.05 Figure 2c Peripheral blood lymphocytes derived from a healthy donor were cultured either in culture medium or in serum. Serum was derived either from an allogeneic healthy donor or from melanoma patients with either low or high percentage of CEACAM1-positive lymphocytes, as indicated in the figure. Peripheral blood lymphocytes from four different donors were tested, each in three different sera samples from each category. The figures show the staining results of gated lymphocytes of a representative experiment.

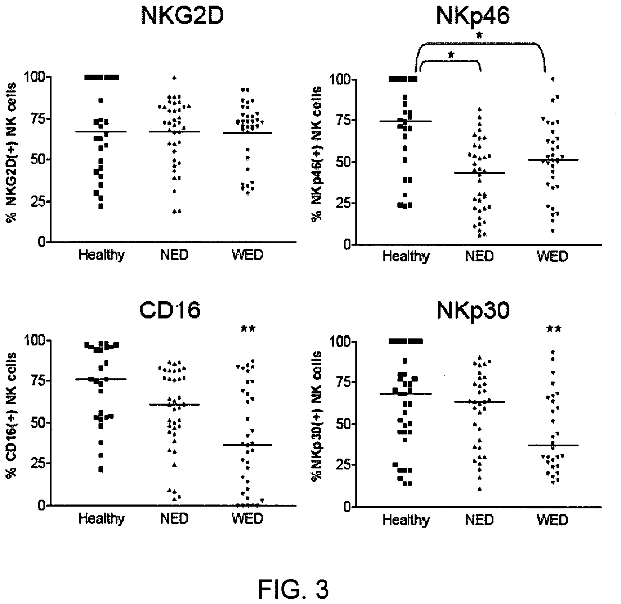

FIG. 3 illustrates dysregulated expression of NK activating receptors on circulating NK cells. The figure shows scatter distribution of various NK activating receptors (indicated in the figure) expression profile on circulating NK cells. There are three main groups of samples, healthy donors (black squares), patients with no evidence of disease (NED, black upright triangles) and patients with evidence of disease (WED, black inverse triangles). Each individual shape represents a single sample from the same group. Y axis denotes the percent of receptor-positive circulating NK cells. Horizontal lines indicate the median value of the group, which is indicated numerically below. Non parametric two sided t-test was used to compare between different groups, as indicated in each plot. * denotes P value < 0.05, ** denotes P value < 0.01.

### DESCRIPTION OF SPECIFIC EMBODIMENTS OF THE INVENTION

The present invention, in some embodiments thereof, relates to methods for diagnosing melanoma.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

The American Cancer Society estimates the lifetime risk that an individual will develop cancer is 1 in 2 for men and 1 in 3 for women. The development of cancer, while still not completely understood, can be enhanced as a result of a variety of risk factors. For example, exposure to environmental factors (e.g., tobacco smoke) might trigger modifications in certain genes, thereby initiating cancer development. Alternatively, these genetic modifications may not require an exposure to environmental factors to become abnormal. Indeed, certain mutations (e.g., deletions, substitutions, etc.) can be inherited from generation to generation, thereby imparting an individual with a genetic predisposition to develop cancer.

Currently, the survival rates for many cancers are on the rise. One reason for this success is improvement in the detection of cancer at a stage at which treatment can be effective. Indeed, it has been noted that one of the most effective means to survive cancer is to detect its presence as early as possible. According to the American Cancer Society, the relative survival rate for many cancers would increase by about 15 % if individuals participated in regular cancer screenings. Therefore, it is becoming increasingly useful to develop novel diagnostic tools to detect the cancer preferably before it develops or at an as early stage of development as possible.

Soluble CEACAM1 has been previously suggested as a tumor marker, especially for melanoma. Interestingly, soluble CEACAM1 is also apparent in the serum of healthy individuals albeit at lower levels.

The present inventor has uncovered that CEACAM1 is expressed on peripheral blood lymphocytes (PBLs) of melanoma affected individuals, while expression of this protein on PBLs of healthy individuals is missing (undetectable using FACS).

This finding suggests that testing CEACAM1 expression on isolated lymphocytes can be effectively used in diagnosing melanoma with reduced erroneously positive readings.

Thus, according to an aspect of the invention there is provided a method of diagnosing melanoma, the method comprising determining a level of CEACAM1 on isolated peripheral blood lymphocytes (PBLs) of a subject in need thereof, wherein an upregulation of said level of CEACAM1 above a predetermined threshold is indicative of cancer in said subject.

As used herein the term "diagnosing" refers to determining presence or absence of a pathology, classifying a pathology or a symptom, determining a severity of the pathology, monitoring pathology progression, forecasting an outcome of a pathology and/or prospects of recovery. The term diagnosis also refers, in some embodiments thereof, to screening. Screening for cancer can lead to earlier diagnosis in specific cases. Early diagnosis may lead to extended life.

As used herein the phrase "subject in need thereof' refers to a human subject who is at risk of having melanoma [e.g., a genetically predisposed subject, a subject with medical and/or family history of melanoma, a subject who has been exposed to carcinogens, occupational hazard, environmental hazard] and/or a subject who exhibits suspicious clinical signs of melanoma. Additionally or alternatively, the subject in need thereof can be a healthy human subject undergoing a routine well-being check up.

As used herein the term "CEACAM1" refers to the mRNA or protein product of the CEACAM1 gene e.g., Nucleotide sequences of CEACAM1 include, but are not limited to, NM_001184816.1 GI:296317313 (SEQ ID NO: 5), NM_001184813.1 GI:296317304 (SEQ ID NO: 6), NM_001184815.1 GI:296317311 (SEQ ID NO: 7), NM_001024912.2 GI:296317301 (SEQ ID NO: 8), NM_001712.4 GI:296317298 (SEQ ID NO: 9). Protein sequences of CEACAM1 include but are not limited to, AAH24164.1 (SEQ ID NO: 10), AAH14473.1 (SEQ ID NO: 11), NP_001171745.1 (SEQ ID NO: 12), NP_001171742.1(SEQ ID NO: 13), NP_001171744.1 (SEQ ID NO: 14), P13688.2 (SEQ ID NO: 15).

As used herein, the phrase "peripheral blood lymphocytes" refers to a sample taken from circulating blood as opposed to blood cells sequestered within the lymphatic system, spleen, liver, or bone marrow. The term refers to large granular lymphocytes and small lymphocytes. Large granular lymphocytes include natural killer cells (NK cells). Small lymphocytes consist of T cells and B cells.

As used herein the term "isolated" refers to isolated from the natural environment. According to a specific embodiment, the term relates to serum purified i.e., no plasma.

Peripheral blood cell samples are typically taken using a syringe with a needle.

Methods of processing peripheral blood cell samples are known in the art and further described in the Examples section herein below.

It will be appreciated that determining the level of CEACAM1 in peripheral blood is performed *ex vivo* (on a sample derived from the subject)..

As used herein, the phrase "level of CEACAM1" refers to the degree of gene expression and/or gene product activity (e.g., inhibition of NK killing activity as shown in Figures2A-C) of the CEACAM1 gene in the biological sample. Accordingly, the level of CEACAM1 can be determined at the amino acid level using protein detection methods.

Thus, the level of the CEACAM1 amino acid sequence (CEACAM1 protein) can be determined using a CEACAM1 specific antibody via the formation of an immunocomplex [*i.e*., a complex formed between the CEACAM1 antigen (a CEACAM1 amino acid sequence) present in the biological sample and the CEACAM1 specific antibody].

The immunocomplex of the present invention can be formed at a variety of temperatures, salt concentration and pH values which may vary depending on the method and the biological sample used and those of skills in the art are capable of adjusting the conditions suitable for the formation of each immunocomplex.

The term "antibody" as used in this invention includes intact molecules as well as functional fragments thereof, such as Fab, F(ab')2, Fv or single domain molecules such as VH and VL to an epitope of an antigen. These functional antibody fragments are defined as follows: (1) Fab, the fragment which contains a monovalent antigen-binding fragment of an antibody molecule, can be produced by digestion of whole antibody with the enzyme papain to yield an intact light chain and a portion of one heavy chain; (2) Fab', the fragment of an antibody molecule that can be obtained by treating whole antibody with pepsin, followed by reduction, to yield an intact light chain and a portion of the heavy chain; two Fab' fragments are obtained per antibody molecule; (3) (Fab')2, the fragment of the antibody that can be obtained by treating whole antibody with the enzyme pepsin without subsequent reduction; F(ab')2 is a dimer of two Fab' fragments held together by two disulfide bonds; (4) Fv, defined as a genetically engineered fragment containing the variable region of the light chain and the variable region of the heavy chain expressed as two chains; (5) Single chain antibody ("SCA"), a genetically engineered molecule containing the variable region of the light chain and the variable region of the heavy chain, linked by a suitable polypeptide linker as a genetically fused single chain molecule; and (6) Single domain antibodies are composed of a single VH or VL domains which exhibit sufficient affinity to the antigen.

Methods of producing polyclonal and monoclonal antibodies as well as fragments thereof are well known in the art (See for example, Harlow and Lane, Antibodies: A Laboratory Manual, Cold Spring Harbor Laboratory, New York, 1988, incorporated herein by reference).

Antibody fragments according to the present invention can be prepared by proteolytic hydrolysis of the antibody or by expression in E. coli or mammalian cells (e.g. Chinese hamster ovary cell culture or other protein expression systems) of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, by Goldenberg, U.S. Pat. Nos. 4,036,945 and 4,331,647, and references contained therein, which patents are hereby incorporated by reference in their entirety. See also Porter, R. R. [Biochem. J. 73: 119-126 (1959)]. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Fv fragments comprise an association of VH and VL chains. This association may be noncovalent, as described in Inbar et al. [Proc. Nat'l Acad. Sci. USA 69:2659-62 (19720]. Alternatively, the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (scFv) are prepared by constructing a structural gene comprising DNA sequences encoding the VH and VL domains connected by an oligonucleotide. The structural gene is inserted into an expression vector, which is subsequently introduced into a host cell such as E. coli. The recombinant host cells synthesize a single polypeptide chain with a linker peptide bridging the two V domains. Methods for producing scFvs are described, for example, by Whitlow and Filpula, Methods 2: 97-105 (1991); Bird et al., Science 242:423-426 (1988); Pack et al., Bio/Technology 11:1271-77 (1993); and U.S. Pat. No. 4,946,778, which is hereby incorporated by reference in its entirety.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry [Methods, 2: 106-10 (1991)].

Antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introduction of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10,: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995).

According to the method of this aspect of the present invention, an amount of immunocomplex formation is indicative of a diagnosis of the cancer. Various methods can be used to detect the formation of the CEACAM1 immunocomplex of the present invention and those of skills in the art are capable of determining which method is suitable for each immunocomplex.

Anti CEACAM1 antibodies are known in the art, some of which are described in the Examples section which follows (see also U.S. Pat. Application 20090181403, which teaches polyclonal or monoclonal antibodies for CEACAM1, herein incorporated by reference in its entirety). Exemplar CEACAM1 specific antibodies include: Mouse monoclonal [29H2] to CEACAM1; Mouse monoclonal [GM8G5] to CEACAM1 (GM8G5 recognizes the Human CEACAM1 A2 domain); CEACAM1 antibody number 2037.00.02 (from Strategic Diagnostics Inc) binding CEACAM1 amino acids 35-134; the CEACAM1-specific antibody 4D1/C2; anti-CEACAMl 5F4 mAb; anti-CEACAMl Kat4c mAb; or any combination or derivative thereof. The CEACAM1 binding agent can also be a member of the CEA protein family. Another antibody which can be used in accordance with the present teachings is such one that has the CDRs of the antibody produced from the hybridoma cell which has been deposited under ATCC Accession Number PTA-9974.

The CEACAM1 antibody used in the immunocomplex of the present invention can be labeled using methods known in the art. It will be appreciated that the labeled antibodies can be either primary antibodies (*i*.*e*., which bind to the specific antigen, e.g., a CEACAM1-specific antigen) or secondary antibodies (e.g., labeled goat anti rabbit antibodies, labeled mouse anti human antibody) which bind to the primary antibodies. The antibody can be directly conjugated to a label or can be conjugated to an enzyme.

Antibodies used in the present invention can be fluorescently labeled (using a fluorescent dye conjugated to an antibody), radiolabeled (using radiolabeled e.g., ¹²⁵I, antibodies), or conjugated to an enzyme (e.g., horseradish peroxidase or alkaline phosphatase) and used along with a chromogenic substrate to produce a colorimetric reaction. The chromogenic substrates utilized by the enzyme-conjugated antibodies of the present invention include, but are not limited to, AEC, Fast red, ELF-97 substrate [2-(5'-chloro-2-phosphoryloxyphenyl)-6-chloro-4(3H)-quinazolinone], p-nitrophenyl phosphate (PNPP), phenolphthalein diphosphate, and ELF 39-phosphate, BCIP/INT, Vector Red (VR), salmon and magenta phosphate (Avivi C., et al., 1994, J Histochem. Cytochem. 1994; 42: 551-4) for alkaline phosphatase enzyme and Nova Red, diaminobenzidine (DAB), Vector(R) SG substrate, luminol-based chemiluminescent substrate for the peroxidase enzyme. These enzymatic substrates are commercially available from Sigma (St Louis, MO, USA), Molecular Probes Inc. (Eugene, OR, USA), Vector Laboratories Inc. (Burlingame, CA, USA), Zymed Laboratories Inc. (San Francisco, CA, USA), Dako Cytomation (Denmark).

Detection of the CEACAM1 immunocomplex in PBCs can be performed using fluorescence activated cell sorting (FACS), enzyme linked immunosorbent assay (ELISA), Western blot and radio-immunoassay (RIA) analyses, immunoprecipitation (IP) with optionally the use of magnetic beads or by a molecular weight-based approach.

For Western blot the proteins are extracted from a cell sample and are subjected to electrophoresis (e.g., SDS-PAGE) and blotting to a membrane (e.g., nitrocellulose or PVDF). The membrane is then interacted with a CEACAM1 antibody which can be either directly labeled or further subjected to a secondary labeled antibody. Detection may be by autoradiography, colorimetric reaction or chemiluminescence. This method allows both quantitation of an amount of substrate and determination of its identity by a relative position on the membrane which is indicative of a migration distance in the acrylamide gel during electrophoresis.

In case the concentration of the antigen in the biological sample is low, detection of the antigen (CEACAM1 amino acid sequence) can be performed by immunoprecipitation (IP). For immunoprecipitation analysis the CEACAM1 antibody may directly interact with a sample (e.g., cell lysate) including CEACAM1 and the formed complex can be further detected using a secondary antibody conjugated to beads (e.g., if the CEACAM1 antibody is a mouse monoclonal antibody, the secondary antibody may be an anti-mouse antibody conjugated to e.g., Sepharose beads). The beads can be then precipitated by centrifugation, following which the precipitated proteins (e.g., CEACAM1 and anti CEACAM1 antibodies) can be detached from the beads (e.g., using denaturation at 95 °C) and further subjected to Western blot analysis using the CEACAM1 specific antibodies. Alternatively, the anti-CEACAM1 antibody and the beads-conjugated secondary antibody may be added to the biological sample containing the antigen (CEACAM1) to thereby form an immunocomplex, followed by Western blot analysis with anti-CEACAMl antibodies.

FACS analysis enables the detection of antigens present on cell membranes such as CEACAM1. Briefly, CEACAM1 specific antibodies are linked to fluorophores and detection is performed by means of a cell sorting machine which reads the wavelength of light emitted from each cell as it passes through a light beam. This method may employ two or more antibodies simultaneously.

The level of CEACAM1 can be also determined using ELISA. Briefly, a sample containing CEACAM1 antigen is fixed to a surface such as a well of a microtiter plate. An antigen specific antibody (a CEACAM1 antibody) coupled to an enzyme is applied and allowed to bind to the antigen. Presence of the antibody is then detected and quantitated by a colorimetric reaction employing the enzyme coupled to the antibody. Enzymes commonly employed in this method include horseradish peroxidase and alkaline phosphatase. If well calibrated and within the linear range of response, the amount of substrate present in the sample is proportional to the amount of color produced. A substrate standard is generally employed to improve quantitative accuracy.

The level of CEACAM1 can be also determined using radio-immunoassay (RIA). In one version, this method involves precipitation of the desired antigen (CEACAM1) with a specific antibody and radiolabeled antibody binding protein (e.g., protein A labeled with I¹²⁵) immobilized on a precipitable carrier such as agarose beads. The number of counts in the precipitated pellet is proportional to the amount of antigen.

In an alternate version of the RIA, a labeled antigen and an unlabelled antibody binding protein are employed. A sample containing an unknown amount of antigen is added in varying amounts. The decrease in precipitated counts from the labeled antigen is proportional to the amount of antigen in the added sample.

The level of CEACAM1 can be also determined using molecular weight-based approach. Since the immunocomplex exhibits a higher molecular weight than its components, methods capable of detecting such a change in the molecular weight can be also employed. For example, the immunocomplex can be detected by a gel retardation assay. Briefly, a non-denaturing acrylamide gel is loaded with samples. A shift in the size (molecular weight) of the protein product as compared with its components is indicative of the presence of an immunocomplex. Such a shift to a higher molecular weight can be viewed using a non-specific protein staining such as silver stain or Commassie blue stain.

It will be appreciated that analyzing an amount of CEACAM1 in PBCs may also be effected at the polynucleotide level. RNA detection methods can be performed using an isolated polynucleotide (e.g., a polynucleotide probe, an oligonucleotide probe/primer) capable of hybridizing to a CEACAM1 nucleic acid sequence such as the CEACAM1 transcript set forth by NM_001184816.1 GI:296317313 (SEQ ID NO: 5), NM_001184813.1 GI:296317304 (SEQ ID NO: 6), NM_001184815.1 GI:296317311 (SEQ ID NO: 7), NM_001024912.2 GI:296317301 (SEQ ID NO: 8), NM_001712.4 GI:296317298 (SEQ ID NO: 8). Examples for such oligonucleotide probe/primer sequences are set forth in SEQ ID NOs:1-3. Such a polynucleotide can be at any size, such as a short polynucleotide (e.g., of 15-200 bases), an intermediate polynucleotide of 100-2000 bases and a long polynucleotide of more than 2000 bases.

The isolated polynucleotide probe used by the present invention can be any directly or indirectly labeled RNA molecule [e.g., RNA oligonucleotide (e.g., of 17-50 bases), an *in vitro* transcribed RNA molecule], DNA molecule (e.g., oligonucleotide, e.g., 15-50 bases, cDNA molecule, genomic molecule) and/or an analogue thereof [e.g., peptide nucleic acid (PNA)] which is specific to the CEACAM1 RNA transcript of the present invention.

Oligonucleotides designed according to the teachings of the present invention can be generated according to any oligonucleotide synthesis method known in the art such as enzymatic synthesis or solid phase synthesis. Equipment and reagents for executing solid-phase synthesis are commercially available from, for example, Applied Biosystems. Any other means for such synthesis may also be employed; the actual synthesis of the oligonucleotides is well within the capabilities of one skilled in the art and can be accomplished via established methodologies as detailed in, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988) and "Oligonucleotide Synthesis" Gait, M. J., ed. (1984) utilizing solid phase chemistry, e.g. cyanoethyl phosphoramidite followed by deprotection, desalting and purification by for example, an automated trityl-on method or HPLC.

The isolated polynucleotide used by the present invention can be labeled either directly or indirectly using a tag or label molecule. Such labels can be, for example, fluorescent molecules (e.g., fluorescein or Texas Red), radioactive molecule (e.g., ³²P-γ-ATP or ³²P-α-ATP) and chromogenic substrates [e.g., Fast Red, BCIP/INT, available from (ABCAM, Cambridge, MA)]. Direct labeling can be achieved by covalently conjugating a label molecule to the polynucleotide (e.g., using solid-phase synthesis) or by incorporation via polymerization (e.g., using an *in vitro* transcription reaction or random-primed labeling). Indirect labeling can be achieved by covalently conjugating or incorporating to the polynucleotide a non-labeled tag molecule (e.g., Digoxigenin or biotin) and subsequently subjecting the polynucleotide to a labeled molecule (e.g., anti-Digoxigenin antibody or streptavidin) capable of specifically recognizing the non-labeled tag.

The above-described polynucleotides can be employed in a variety of RNA detection methods such as Northern blot analysis, reverse-transcribed PCR (RT-PCR) [e.g., a semi-quantitative RT-PCR, quantitative RT-PCR using e.g., the Light Cycler™ (Roche)], RNA *in situ* hybridization (RNA-ISH), *in situ* RT-PCR stain [e.g., as described in Nuovo GJ, et al. 1993, Intracellular localization of polymerase chain reaction (PCR)-amplified hepatitis C cDNA. Am J Surg Pathol. 17: 683-90, and Komminoth P, et al. 1994, Evaluation of methods for hepatitis C virus detection in archival liver biopsies. Comparison of histology, immunohistochemistry, in situ hybridization, reverse transcriptase polymerase chain reaction (RT-PCR) and in situ RT-PCR. Pathol Res Pract., 190: 1017-25] and oligonucleotide microarray analysis [e.g., using the Affymetrix microarray (Affymetrix®, Santa Clara, CA)].

As mentioned, a level of CEACAM1 in a PBC sample above a predetermined threshold is indicative of the cancer.

The "predetermined threshold" may be experimentally determined by comparing normal PBC samples (e.g., samples obtained from healthy subjects, not affected with cancer) to PBC samples derived from subjects known to have carcinogenesis such as CRC. Preferably, a statistically significant number of samples are analyzed.

It will be appreciated that the presence of melanoma can be further validated using additional assays. For example, in case the level of CEACAM1 detected in a PBC sample of a subject is above a predetermined threshold, additional assays such as Gold-standard assays including but not limited to histology, imaging, molecular markers, blood tests followed by histological evaluations (including CEACAM1 immunostaining), the "ABCDE" and skin biopsy may be performed.

Once results are obtained the subject is informed of the test results i.e., presence or absence of melanoma and suitable treatments may be initiated.

Diagnostic compositions may, if desired, be presented in an article of manufacture e.g., kit, such as an FDA approved kit, which may contain diagnostic reagents and instructions for use.. The kit may also be accommodated by a notice associated with the container in a form prescribed by a governmental agency regulating the manufacture, use or sale of pharmaceuticals, which notice is reflective of approval by the agency of the form of the compositions or human or veterinary use.

The terms "comprises", "comprising", "includes", "including", "having" and their conjugates mean "including but not limited to".

The term "consisting of means "including and limited to".

The term "consisting essentially of' means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### EXPERIMENTAL PROCEDURES

### Patients and controls

Patients with pathologically verified cutaneous malignant melanoma in all AJCC stages of disease were included. There were no exclusion criteria. Patients were broadly categorized clinically into two groups: a) patients with no evidence of disease (NED) at the time of blood sampling that were further subdivided into low risk of recurrence (AJCC stage I or II) and high risk of recurrence (AJCC stage III or IV); b) patients with evidence of active disease (WED) at the time of blood sampling, who were subcategorized according AJCC criteria. High risk NED patients (AJCC stages III and IV) have received prior therapy that yielded disease regression. All normal controls were in excellent health at the time of the study. All melanoma patients and healthy volunteers gave written informed consent prior to their participation in this study. This study was approved by the Sheba Medical Center Institutional Review Board.

### Specimen characteristics

Blood samples were obtained from healthy individuals and patients by veno-puncture (> 3 ml)and standard handling procedures. Peripheral blood lymphocytes were purified using a density gradient and deep frozen in liquid nitrogen. Anonymous samples (marked only with ID number) were linked only to clinical-pathological data.

### Study design

Blood was obtained in the melanoma clinic with no case selection. None of the patients underwent surgery near the time of blood sampling. Study was retrospective: a single blood sample was obtained from all patients, frozen and analyzed at a later, technically convenient, point. Each sample was tested two independent times in triplicate repeats. Follow up in this study began from time of blood sampling. The mean follow up time was 12 months all groups, except for stage IV-M1c WED patients, due to death of some of the patients shortly after blood sampling (Table 1). The clinical endpoints examined were disease free period (DFP) for NED patients and survival for WED patients.

### Antibodies

Antibodies directed against CEACAM proteins included in this work were: murine anti-human CEACAM1 monoclonal antibodies NC8 [Albarran-Somoza B, Franco-Topete R, Delgado-Rizo V et al (2006) CEACAM1 in cervical cancer and precursor lesions: association with human papillomavirus infection. J Histochem Cytochem. 54: 1393-1399], murine anti-human CEACAM1, 5, 6, 8 monoclonal antibody Kat4c (Dako, Glustrup Denmark) and purified rabbit polyclonal anti-human CEACAM1, 5, 6 antibodies (Dako, Glustrup Denmark). The following conjugated monoclonal antibodies were used: anti human CD3-FITC (IQ); anti human CD56-PE/Cy5.5 (eBiocience); anti human NKp46-APC (eBiocience); anti human NKp30-APC (eBiocience); anti human CD16-PE (eBiocience); anti human NKG2D-APC (R&D Systems, Minneapolis, MN, USA), biotinylated NC8 and biotinylaated rabbit polyclonal anti-human CEACAM1, 5, 6 antibodies. Secondary reagents included PE-conjugated F(ab')2 fragments of goat anti human-Fc IgG (Jackson ImmunoResearch); FITC-conjugated F(ab')2 fragments of goat anti mouse-Fc IgG (ICN) and PE-conjugated streptavidin (Jackson Immunoresearch). Biotinylation of antibodies was performed with SS biotin (Chemicon) according to manufacturer's instructions.

### Flow cytometry

Binding of antibodies to cells was tested in standard flow cytometry procedures as formerly reported [Markel G, Seidman R, Stern N et al (2006). Inhibition of human tumor-infiltrating lymphocyte effector functions by the homophilic carcinoembryonic cell adhesion molecule 1 interactions. J Immunol 177: 6062-6071; Markel G, Seidman R, Cohen Y et al 2009 Feb;126(2):186-200. Epub 2008 Jun 13 Dynamic expression of protective CEACAM1 on melanoma cells during specific immune attack. PBLs are characterized as having small Forward and Side Scatter value in flow cytometry, and were gated accordingly. The cells were stained with a mixture of antibodies, including CD3 (T cell marker), CD56 (NK marker) and an antibody for CEACAM1 (e.g. Kat4c). T cells were defined as CD3-positive CD56-negative lymphocytes (within the Forward & Side Scatter gate described above). NK cells were defined as CD3-negative CD56-positive lymphocytes (within the Forward & Side Scatter gate described above),

### RESULTS

### Unusually high percentage of CEACAM1-positive NK and T cells in the peripheral blood of melanoma patients

CEACAM1 expression pattern was determined on gated NK and T cells derived from peripheral blood lymphocytes of healthy donors and the melanoma patients. The mean percentage of CEACAM1-positive NK cells in healthy donors was 15% (Figure 1a). A significantly enhanced proportion of CEACAM-positive NK cells (33%) was observed in NED patients, but the highest proportion (45%) was observed in WED patients (Figure 1a). Similarly, a significant increase in the mean proportion of CEACAM1-positive T cells (31%) was observed in WED patients (Figure 1b). The percentage of ceacam + in t cells of NED was lower than 5. A statistically significant positive correlation between CEACAM1 expression by NK and T cells could be observed in WED patients (Spearman's r = 0.5, P value < 0.05). A similar, yet milder, trend was observed in NED patients, without reaching statistical significance (Spearman's r = 0.267, P value = 0.082). There was no correlation between percentages of CEACAM1 either on T or NK cells with the serum concentration of CEACAM1. In conclusion, although both soluble CEACAM1 concentrations and CEACAM1 expression on lymphocytes are generally linked to disease activity, they are not connected directly to each other

Indeed, when WED patients were further categorized into DOD (died of disease) and AWD (alive with disease) patients, the mean proportion of CEACAM1-positive NK cells among DOD patients (51.9%) was significantly higher than in AWD patients (34.4%) (Figure 1c). However, there was no clear correlation between the percentage of CEACAM1 expression and time-to-death among these patients (data not shown). There was no difference in percentage of CEACAM1-positive T cells between DOD and AWD patients (Figure 1d).

### Enhanced CEACAM1 expression is functional and inhibits NK-mediated lysis

Peripheral blood lymphocytes were derived either from melanoma patients (exemplar patients 38 and 71) or from healthy donors (Figure 2a). CEACAM1-mediated inhibition of fresh lymphocytes was tested in natural cytotoxicity assays. The NK-sensitive 721.221 (.221/Mock) and 721.221 stably transfected with the CEACAM1 protein (.221/CEACAM1) were used as target cells. Natural killing activity of .221 cells was clearly observed with lymphocytes derived from all sources (Figure 2b). Remarkably, a moderate, yet reproducible and significant inhibition of killing of the .221/CEACAM1 cells was observed only with the patient-derived lymphocytes (Figure 2b). No similar inhibition was measured with the healthy donor derived lymphocytes (Figure 2b). Similar results were observed with lymphocytes derived from other patients (data not shown) as well as in re-directed lysis experiments performed with concurrent CEACAM1 engagement (data not shown). These results show that enhanced CEACAM1 expression on circulating NK cells is functional, and may expose the patient's immune system to CEACAM1-mediated inhibition.

### Sera from patients do not induce CEACAM1 expression on lymphocytes

Fresh peripheral blood lymphocytes from healthy donors were incubated for 48 hours in culture medium or serum derived either from: healthy donors, patients with low percentage of CEACAM1-positive lymphocytes or patients with high percentage of CEACAM1-positive lymphocytes. CEACAM1 was analyzed on gated lymphocytes cells. There were no significant differences in the expression of CEACAM1 among the different treatments on either CD56(+) or CD56(-) cells **(****Figure 2c**). These experiments suggest that the high expression of CEACAM1 on lymphocytes observed in melanoma patients is probably not due to systemic soluble factors.

### The phenotype of circulating NK cells in melanoma patients is generally abnormal

Peripheral blood NK cells were stained for the expression of various killing receptors, including NKG2D, NKp46, CD16 and NKp30. A remarkable decrease was observed in the expression profiles of NKp46, CD16 and NKp30, but not in NKG2D. Specifically, NKp46 was significantly downregulated among all patients, as compared with healthy donors, but there was no significant difference between NED and WED patients. A significant downregulation of CD16 and NKp30 was observed among WED patients, as compared to NED patients and healthy donors. There were no significant differences between NED patients and healthy donors in the expression of these receptors (Figure 3). A statistically significant positive correlation was identified between CD16, NKp30 and NKp46, and in additional, between NKp46 and NKG2D (Table 1, below). A striking negative correlation was evident between expression of CEACAM1 and the expression of all killing receptors tested, except NKG2D (Table 1). These results indicate on a systemic irregularity in NK cell phenotype, which is not confined only to CEACAM1 expression.

**Table 1: Correlation between CEACAM1 expression and NK activating receptors in melanoma patients**

| The correlation was calculated using Spearman's test. Table summarizes Spearman's R values between each pair of parameters. * denotes P value < 0.05, ** denotes P value < 0.01, *** denotes P value < 0.001. | | | | | |
|---|---|---|---|---|---|
| **NKG2D** | **NKp46** | **NKp30** | **CD16** | **CEACAM1** | |
| -0.024 | -0.270* | -0.437*** | -0.328** | 1 | **CEACAM1** |
| 0.13 | 0.376** | 0.414** | 1 | -0.328** | **CD16** |
| 0.072 | 0.353** | 1 | 0.414** | -0.437*** | **NKp30** |
| 0.351** | 1 | 0.353** | 0.376** | -0.207* | **NKp46** |
| 1 | 0.351** | 0.072 | 0.13 | -0.024 | **NKG2D** |

## Claims

1. A method of diagnosing melanoma, the method comprising determining a level of CEACAM1 on isolated peripheral blood lymphocytes (PBLs) of a subject in need thereof, wherein an upregulation of said level of CEACAM1 above a predetermined threshold is indicative of melanoma in said subject, wherein said PBLs comprise T cells or NK cells.

2. The method of claim 1, wherein said PBLs comprise T cells.

3. The method of claim 1, wherein said PBLs comprise NK cells.

4. The method of claim 1, wherein said determining a level of CEACAM1 on isolated peripheral blood lymphocytes (PBLs) is effected by FACS.

5. The method of claim 1, further comprising informing the subject on the presence or absence of the melanoma or stage thereof.

6. The method of claim 1, further comprising validating the diagnosis or prognosis using a method selected from the group consisting of surgical biopsy, imaging, pathology and molecular testing.

## Patentansprüche

1. Verfahren zur Diagnose von Melanom, wobei das Verfahren umfasst, Bestimmen einer Menge CEACAM1 an isolierten peripheren Blut-Lymphozyten (PBLs) eines betreffenden Individuums, wobei ein Anstieg der Menge an CEACAM1 über einen bestimmten Grenzwert Melanom in dem Individuum anzeigt, worin die PBLs T-Zellen oder NK-Zellen umfassen.

2. Verfahren nach Anspruch 1, wobei die PBLs T-Zellen umfassen.

3. Verfahren nach Anspruch 1, wobei die PBLs NK-Zellen umfassen.

4. Verfahren nach Anspruch 1, wobei das Bestimmen einer Menge an CEACAM1 an isolierten peripheren Blut-Lymphozyten (PBLs) mittels FACS erfolgt.

5. Verfahren nach Anspruch 1, weiter umfassend, Informieren des Individuums über die Anwesenheit oder Abwesenheit des Melanoms oder über dessen Stadiums.

6. Verfahren nach Anspruch 1, weiter umfassend, Validieren der Diagnose oder Prognose unter Verwendung eines Verfahrens ausgewählt aus der Gruppe bestehend aus chirurgischer Biopsie, Abbilden, Pathologie und molekularer Untersuchung.

## Revendications

1. Procédé de diagnostic du mélanome, le procédé comprenant la détermination d'un niveau de CEACAM1 sur des lymphocytes sanguins périphériques (LSP) isolés d'un sujet qui en a besoin, une régulation à la hausse dudit niveau de CEACAM1 au-dessus d'un seuil prédéterminé étant indicatrice du mélanome chez ledit sujet, lesdits LSP comprenant des lymphocytes T ou des cellules NK.

2. Procédé selon la revendication 1, lesdits LSP comprenant des lymphocytes T.

3. Procédé selon la revendication 1, lesdits LSP comprenant des cellules NK.

4. Procédé selon la revendication 1, ladite détermination d'un niveau de CEACAM1 sur des lymphocytes sanguins périphériques (LSP) isolés étant effectuée par FACS (fluorescence activated cell sorting - tri cellulaire activé par fluorescence).

5. Procédé selon la revendication 1, comprenant en outre l'information du sujet sur la présence ou l'absence du mélanome ou du stade de celui-ci.

6. Procédé selon la revendication 1, comprenant en outre la validation du diagnostic ou du pronostic à l'aide d'un procédé choisi dans le groupe constitué par une biopsie chirurgicale, une imagerie, une pathologie et un test moléculaire.
